# EUROPEAN PATENT SPECIFICATION

(11) **EP 4 547 063 B1**
(45) Date of publication and mention of the grant of the patent: **04.11.2026**
(21) Application number: 23736487.2
(22) Date of filing: 29.06.2023
(51) Int. Cl.: A47D 9/02, A47D 15/00

(54) **DEVICE, SYSTEM AND METHOD FOR ENCOURAGING SLEEP OF A CHILD**
VORRICHTUNG, SYSTEM UND VERFAHREN ZUR FÖRDERUNG DES SCHLAFS EINES KINDES
DISPOSITIF, SYSTÈME ET PROCÉDÉ POUR ENCOURAGER UN ENFANT À S'ENDORMIR

(30) Priority: 30.06.2022 NL 2032343
(43) Date of publication of application: 07.05.2025
(73) Proprietor: Spylo B.V., 2023 VP Haarlem (NL)
(72) Inventor: TEEUWEN, Jeroen Eugene Anton, 2023 VP Haarlem (NL)
(74) Representative: V.O.
(86) International application number: PCT/NL2023/050357
(87) International publication number: WO 2024/005639

(56) References cited:
- US-A1- 2019 117 152
- US-A1- 2022 167 930
- US-B2- 7 849 541

## Description

The invention relates to a device for encouraging sleep of a child. The invention especially relates to such device for encouraging babies or toddlers to sleep.

It is desirable that babies and toddlers have continuous periods of time that they are asleep. Not only is this important to the babies or toddlers themselves, but also to their parents or other carers. If a baby or toddler has difficulty in falling asleep or staying asleep, this can wake up or keep awake a parent or other carer too. This can lead to sleep deprivation for both the baby or toddler and their carers. Sleep deprivation is unhealthy and will lead to for example general tiredness, bad temper, lack of concentration and other sleep related problems. Moreover when a baby or toddler wakes up it may start crying, which in turn may wake up a parent or other people and keep such persons awake or prevent them from doing other things because the baby or toddler needs attention.

It is known that a baby or toddler may be encouraged to fall asleep, by calming the baby or toddler or soothing him or her by resting the baby or toddler against another person's body and / or by simulating the conditions, such as noises or movement, of when the baby was in the womb. This seems especially true when the baby or toddler is held against another person's chest and/or when said person is moving around with the child held against their chest, such that it can experience the breathing of that person. Feeling the other person's chest heave and fall with breathing will be soothing to the child and encourage sleep, both when a child is asleep, maintaining the child's sleep, and to encourage the child to fall asleep. Encouraging sleep can also be encouraging a child to stop crying or not start crying.

Having another person support a child in order to have the child experience the other person's breathing means that the said person has to be close to the child, and will normally keep the person from undertaking other activities, such as for example sleeping, working or the like. This may be burdensome on such person. Moreover, the child may get used to this way of treatment, which will make it more difficult for the baby or toddler to rest and/or sleep without such person being near.

For these reasons several attempts have been made to provide for devices simulating an adults breathing pattern, for encouraging a baby or child to fall asleep.

### BACKGROUND TO THE INVENTION

GB 235994 A discloses a device for simulating a mother's heartbeat to a child lying in a bed. The device comprises a bladder that can be disposed near or at least partially under the child by incorporating it in a specially designed mattress, by placing it on the mattress or by incorporating the bladder in a relatively thin sheet for use on a mattress. An air pressure pulse generator, which is connected to the bladder by a flexible tube, generates rhythmical air pressure pulses according to a pre-set regime. The generator is preferably placed outside the bed.

DE 20205187 U1 discloses a mattress for babies and toddlers which contains a lifting element which, with an area that is smaller than the contact area of the child lying on the mattress, provides a continuously alternating lifting and lowering movement in the breathing rhythm of an adult person to create the illusion that the baby or toddler is lying on the body of the mother or father. The lifting element is an expansion body that expands when a pressure fluid is pumped into the expansion body by a motor-driven pump. The pump and motor are either in or outside of the mattress. In an embodiment inside a mattress a relatively small volume expansion body is used, which is overlaid by a larger plate to have a large enough area going up and down. Correspondingly, another plate is located below the expansion body.

The document US 2019/117152 A1 discloses a system according to the state of the art.

Although these devices can be helpful to encourage babies or toddlers to fall asleep or stay asleep, these devices do have their drawbacks. These devices are relatively complex in construction and use, may require special mattresses and will necessitate active involvement of a carer to activate the device, which for example means that such carer will already have been woken up by a child's crying, or have been forced away from an activity the carer was undertaking. Furthermore, safety is an important issue in this type of device. Hence, there is a need for an alternative device for encouraging sleep and/or improve resting of a child or toddler.

### SUMMARY OF THE INVENTION

In an aspect as recited in claim 1, a device for a cot or child's bed to encourage sleep, comprises a support device to be placed in or under a mattress, which support device comprises at least one plank and at least one support. Moreover a lifting unit is provided which comprises at least one bladder, preferably at least two bladders, positioned between the at least one plank and the at least one support. The lifting unit further comprises a control unit which is configured to inflate and/or deflate the at least one bladder via a pomp of a pump system for providing an alternating upward and downward motion of the at least one plank. Moreover a pliable skirt extending from a circumference of the at least one plank is provided. Said upward and downward motion can be experienced by the at least one plank moving, especially tilting up and down with the inflating and deflating of the bladder. Therefore a baby or toddler resting with at least a part of the body, especially an upper body, including a torso and head, on a mattress positioned over the device will experience the said up and down movement. The device may simulate a heartbeat and/or calming noise, thereby creating a natural sense of safety, connection and relaxation for the child or toddler.

By providing a skirt, it can be prevented that a toddler or child, or any person for that matter, can get their fingers stuck between the at least one plank and the at least one support or the at least one plank and a cot or child's bed. By having a pliable skirt, as opposed to a rigid or stiff skirt, it is possible to accommodate for a large range of cots or child's beds, as it can be possible to fold or otherwise adapt the shape of the pliable skirt accordingly.

The phrase 'to encourage sleep' should be interpreted as directly encouraging sleep, but also indirectly, for example by allowing the toddler or child to relax or come to rest significantly, such that the toddler or child for example has an easier time to fall asleep.

In embodiments of a device according to the disclosure, the skirt can be connected to the circumference of the one plank. The skirt can be provided connected to the circumference of the one plank, forming a more robust whole than for example if the skirt is provided separately. The skirt can be connected permanently, for example by using an adhesive or mechanical connecting arrangements such as nails, or semi-permanently for example using a zipper or click systems. The skirt can be an integral part of at least one of the at least one plank and the at least one support.

In embodiments of a device according to the disclosure the skirt can be connected to a circumference of the at least one support, preferably both to the at least one plank and the at least one support. This can further prevent access, for example via the at least one support or between the at least one support and the skirt, thereby further preventing fingers or other body parts to get stuck between the at least one plank and the at least one support or the at least one plank and the child's bed or cot.

In embodiments of a device according to the disclosure the skirt can be suspended from the plank.

In embodiments of a device according to the disclosure the skirt can extend around the perimeter of the plank forming a closed loop. A closed loop can limit access to the space between the at least one plank and at least one support from all sides of the cot or child's bed, further improving safety by reducing the risk of getting fingers or other body parts stuck between moving components.

In embodiments of a device according to the disclosure the skirt can extend elastically in at least an outward direction. By extending outwardly, it can be prevented that the skirt is moved into the space between the at least one plank and the at least one support, thereby limiting the movement of the at least one plank when deflating the at least one bladder. This may also further prevent the risk of for example fingers of a person to get caught between the support and the plank.

In embodiments of a device according to the disclosure the support can be a further plank, comprising a closeable opening for wires and/or tubes. The opening can allow for relative easy placement of the wires, tubes and/or hoses needed to inflate and deflate the at least one bladder. By making the opening closeable, it can be possible to clamp the wires, tubes and/or hoses such that during use their movement is limited. Moreover, by closing off the opening substantially during use, access to the inner space of the device can further be prevented.

In embodiments of a device according to the disclosure the lifting unit can more preferably comprise at least four bladders, wherein at least one bladder can be provided at a head-end of the cot or child's bed during use. By using at least four bladders, especially when placed spaced apart in sets, close to the opposite ends of the plank, a wide range of motions of the at least one plank can be achieved. For example, by inflating and deflating two bladders located at the head end of a child's bed and inflating and deflating two bladders located at an opposite feet end, in opposite phase as the two bladders provided at the heads end, a rocking around a virtual axis perpendicular to the longitudinal axis of the bed can be achieved However, if two bladders on the left side of the bed are inflated and deflated, in opposite phase of two bladders provided on the right side of the bed, a rocking around a virtual axis parallel to the longitudinal axis of the bed can be achieved.

In embodiments of a device according to the disclosure during use at least a minimum pressure is maintained in the or each bladder when deflated, which minimum pressure is above environmental pressure around the pump, such that the relevant bladder remains inflated to at least some extend. By maintaining at least a minimum pressure or pretension in the at least one bladder a response time for the bladder for moving the plank upward at the location of the relevant bladder can be reduced, increasing the system responsiveness. Furthermore, by having the at least one plank rest, or be carried, on the at least one bladder, the location and movement of the child can be determined by measuring pressure changes in the at least one bladder. Preferably the said at least one plank during use is supported only by the bladders. Thus the position of and possible movement of a child or toddler resting on the plank can be measured by pressure changes in said bladders.

In embodiments of a device according to the disclosure each bladder is individually inflatable by a single pump. Having each bladder individually inflated and deflated by a single pump, further control can be achieved over the motion the bladders cause the at least one plank to make during use.

In embodiments of a device according to the disclosure the pump system can comprise a distribution unit, arranged to selectively connect a chosen pump or pump chamber of the pump system to at least one chosen bladder. By making it possible to control which pump or pump chamber actuates which bladder, an even further range of motions can be reached. For example, during use a pump or pump chamber might be switched to activate a different bladder, or switched off altogether. In embodiments of a device according to the disclosure the pump can be connected to the at least one bladder via a hose, wherein the hose between the pump and the at least one bladder has a length such that the pump can be provided outside of the cot or child's bed when in use. The pump can be placed outside the bed, which can allow for easier access for maintenance or control or limit access from a child or toddler located in the bed to the pump by moving it out of reach. Obviously such a hose can in embodiments be provided for each bladder individually.

In embodiments of a device according to the disclosure the pump can comprise at least one pump chamber and a spring, wherein the spring can be arranged to store kinetic energy when the pump chamber expands and to release kinetic energy when the pump chamber contracts.

In embodiments of a device according to the disclosure the pump system can comprise at least one motor arranged to engage at least two pump chambers of the pump. Having a single motor actuate at least two pump chambers, the amount of motors can be limited while still allowing each bladder to be actuated individually.

In embodiments of a device according to the disclosure the pump system can be arranged to form a substantially closed system with the at least one bladder such that during inflation of the at least one bladder an amount of air can be transported towards the at least one bladder that is substantially equal to the amount of air returned to the pump system during deflation of the at least one bladder. In embodiments with multiple bladders and multiple pump chambers, each combination of a pump chamber and the or each bladder connected thereto can form an individual closed system as discussed here before. In further embodiments, each pump chamber may be provided with an over- and/or under pressure valve. The valve can selectively provide a connection of the surrounding of the pump system with the pump chamber, in case the pressure in the corresponding pump chamber is too high, i.e. an over pressure, or if the pressure in the corresponding pump chamber is too low, i.e. near vacuum conditions. The over- and/or under pressure valve can provide for an additional safety measure, both protecting the child from harm due to malfunctioning of the device, or protecting the device from damage. Moreover the over-pressure and/or under-pressure can ensure even better a proper movement pattern of the at least one plank by controlling the pressure and air volume entered into and removed from the bladders.

In embodiments of a device according to the disclosure, the device can further comprise a sensor unit arranged for detecting change in a body-related parameter of the child, wherein the sensor unit further can be configured to activate the control unit when detecting the change in the body-related parameter. Thus the control unit can be activated based on a change in a body related parameter such as for example when a baby starts crying or when a respiratory pattern or change therein, a movement pattern or a change therein or a respiratory pattern or a change therein or combinations thereof is sensed by the sensor unit. This means that with a device according to the disclosure the inflation and deflation of the bladder can be started by the control unit, based on at least one sensor signal, without the necessity of involvement of a carer. Advantageously the device may also monitor if the body-related parameters change in a manner indicating that the infant or toddler resting on the device, is in danger, for example but not limited to a change or stop in respiration of the child or toddler, or an extended period of time during which no motion is detected of the child or toddler. The device may actuate an alarm or may otherwise notify a carer, e.g. by sending an alert to a controlling device such as a smartphone or tablet, that the body-parameters are changing such that the child or toddler may be in peril. Additionally or alternatively the operation of the device can be started and/or stopped differently, for example by a timer or by operation by a user, such as operation of an on-off switch, a digital application such as an app, voice control or the like.

In embodiments of a device according to the disclosure, the sensor unit can comprise a sensor element for sensing a change in the body related parameter, wherein the body-related parameters can for example be at least one of a respiratory rate of the child, a heart rate of the child, sound produced by the child, position of the child and/or movement of the child.

In a device according to the disclosure the each sensor element can be in direct fluidum connection with a pumping chamber of the pump system.

In a device according to the disclosure the at least one plank and the at least one support are preferably relatively stiff, such that with a relatively small volume bladder or combinations of bladders, for example having a volume of below 1 liter, or below 0.5 liter, for example about 200 ml, the at least one plank can be moved relative to the at least one support enough for the child or toddler resting on the mattress to experience the rhythm of heaving and falling induced by the bladder or bladders.

Relatively stiff should at least be understood as meaning that if a child or toddler for which the device is designed lies with his or her upper body part, i.e. torso and head, on the at least one plank, near a side edge thereof, the side edge can still be pushed away from the at least one support by the bladder. For babies and toddlers for example the at least one plank can in embodiments be stiff enough not to allow bending of the relevant side edge over more than about 1 cm when the upper torso of such baby or toddler lies primarily at said side edge. Stiff or at least relatively stiff in this disclosure is to be understood as meaning that if the plank is supported at two opposite longitudinal ends thereof, at a head end and a feet end, in a horizontal position, and a vertical force is exerted to the middle of the plank, i.e. midway between the head end and the feet end, which force is 150 N, the middle of the plank will be pushed down no more than 10% of the distance between the said feet end and the said head end. Preferably the middle of the plank will not be pushed down over more than 6%, preferably not more than 4%, preferably no more than 3%, more preferably no more than 2% such as for example between 0 and 1.5% of said distance.

Preferably as a fluidum for inflating the at least one bladder air is used. Alternatively a fluid such as water can be used, but this may be less desirable since it may leak out of the system, which could lead to wetting of the child's or toddlers cot and could lead to malfunction, especially short circuiting. Moreover, using a fluid like water will make changes in movements and movement directions slower and will need more complicated pump systems than air for similar movements.

Additionally, a relatively stiff at least one plank and at least one support allow for the child or toddler to still move upward, regardless of the position on the mattress and without being in danger of being trapped between a side wall of the bed and the mattress. If the bladder is positioned closer to a hinging axis, a higher upward motion is created than when the bladder is positioned further from the hinging axis. Hence, the position of the bladder may determine the height with which the child or toddler is moved upward and downward. To this end the at least one bladder may be displaceable relative to the plank and/or support.

In embodiments of a device according to the disclosure, the sensor element can be placed in and/or on the lifting unit and/or the support device. In embodiments provide with a sensor unit there may be one or more sensor elements, such as a sound sensor, occupancy sensor, pressure sensor, optical sensor and/or a light sensor that can sense one or more body-related parameters. Moreover, the sensor element can be integrated in the device, but also be or at least comprise a separate element placed close to the child or toddler, such as on the mattress, next to a pillow, hanging from a bedside frame, placed on a nightstand and/or be on the child or toddler such as a wearable device e.g. on the wrist, across the chest or integrated in a piece of clothing, and/or be located outside of the bed such as an occupancy sensor positioned on a wall of a room. Advantageously, a specific sensor element is located such that it can sense a specific body-related parameter best and with the least interference, e.g. when a pressure sensor is placed on the support device and/or in a pump system, it can detect movement of the child or toddler using the device or when a sound sensor is placed on a wall it gets no interference of blankets brushing against the sensor.

Further, in embodiments, a device according to the disclosure may be configured to deactivate after a predetermined time period after activation. When the device is activated and for instance provides a rhythmical upward and downward motion and/or produces sound to simulate the environment of a womb, the device can deactivate after the child or toddler has fallen asleep. This predetermined period may be based on experience of the carer, but can also be based on data collected by the device in which the device itself learned to predict after how long a period of time the child or toddler falls asleep. By deactivating the device after a predetermined time period, the device may use less energy or it may be prevented that the child or toddlers starts waking up again due to the motion, thereby having a counteractive effect.

In embodiments having a sensor unit, a device according to the invention may be configured to activate when a change in the body-related parameter exceeds a predefined threshold value and can be configured to deactivate when the change in the body-related parameters comes below a predefined threshold value. The threshold value may be determined by the user of the device and/or by the device itself by for example using a database created by collecting data on one or more body-related parameters over a period of time. If for instance a baseline is created for the respiratory rate and/or heartrate during sleep, the device may detect how large the deviation from the baseline during sleep is when the child or toddler is waking up and choose this value as a threshold value. Similarly, a baseline may be created for when the child or toddler is awake to notice a change when the child or toddler falls asleep. By having a device that activates and deactivates based on crossing the predetermined threshold value, the device may react before the child or toddler wakes up and/or may be prevented from staying active while the child or toddler has fallen asleep, without the necessity of involvement of a carer.

A device according to the disclosure comprises a support device, wherein the support device may comprise a left portion and a right portion, wherein the left portion and the right portion can be adjustable relative to each other in a direction perpendicular to the direction in which the at least one plank moves relative to the at least one support, for adaption of the width of the support device. By adjusting the support device, the device may be made to fit many different types of beds, like cradles, cods and children's beds, making it more likely that the child or toddler is moving upward and downward even when not lying along the longitudinal centerline of the bed. The support device may be placed in, on or under a mattress and may easy fit in different (baby) beds to simulate the parents breathing by the lifting mechanism. Additionally, a user may not be required to purchase a new device when wanting to use it for another bed than initially acquired for.

Further, to an aspect of the invention the at least one plank or a part thereof may be provided, for example hingeably connected to the at least one support or to a further part of the at least one plank. The or each bladder may be placed between the at least one plank and the at least one support of the support device. The hingeable connection may enable an upward tilting motion of the at least one plank when the bladder is inflated and a downward tilting motion when the bladder is deflated, at least mostly preventing sideward tilting. Said tilting motion may simulate for a child or toddler resting on the device the safe rhythm of a mothers heartbeat and/or womb. The tilting movements can be obtained for example by a bladder placed between the at least one plank and at least one support, by inflating and deflating the bladder, or by other means, for example mechanical means such as for example by a mechanical or electro mechanical actuator, or magnetically.

In a further aspect of the disclosure the at least one plank is hingedly connected to the at least one support, such that the at least one plank can hinge relative to the at least one support, but cannot move about a virtual axis perpendicular to the at least one support during use of the device in a bed.

In an aspect of the disclosure the system can be designed such that the at least one bladder can be held in a fully or at least partly inflated position over a longer period of time. With such partially or fully inflated position the at least one plank can be held in a tilted upward position for a period of time, for example during a period of rest of a child or toddler. Such position can for example be advantageous if it is desired that the child or toddler rests or even sleeps with his or her upper body tilted upward, for example when experiencing reflux, coughing, respiratory difficulties or the like. In embodiments the system, for example the control unit of the system can comprise a switch, such as a mechanical or software switch, for choosing such setting, which can for example be referred to as a reflux setting. In such setting in embodiments it can still be possible to inflate and deflate the bladder as described here before, but then preferably without emptying the bladder fully, such that even during such reciprocal inflation and deflation the at least one plank stays tilted. Alternatively at least two bladders could be used, one for the reflux setting as such and one for the reciprocal movement of the at least one plank.

According to another aspect of the invention, the at least one plank is arranged for hinging upward about a hinging axis and the at least one support comprises a first portion extending from the hinging axis in a first direction that is perpendicular to the hinging axis, to below the at least one plank, and a second portion extending from the hinging axis in a direction opposite to the first direction. The at least one support may extend further along the longitudinal centerline of the bed than the at least one plank to create increased stability. On the portion of the at least one support that extends beyond the hinge and where no at least one plank is present, the mattress and/or the weight of the child or toddlers torso or lower body may push the at least one support towards a bottom portion (e.g. a bed bottom), thereby making it less likely that the support device will tilt about an axis parallel to the longitudinal centerline of a bed.

In embodiments, the device may comprise a speaker unit configured for generating a sound for a predetermined time period upon activation of the control unit by the sensor unit. The speaker unit may for instance be active during the time the lifting mechanism is active, but also for a longer or shorter time period. The speaker unit can produce a sound, such as a voice of a parent, white noise or pink noise (sounds simulating the environment of a womb), a person's heartbeat, for example as recorded from a parent or other carer, or a song. The sound may provide additional comfort to the child or toddler and may encourage falling asleep and/or staying asleep without involvement of a carer. Alternatively, the speaker unit may also be activated and deactivated by a carer, such as a parent, using the control unit or a controlling device such as a tablet, laptop or smartphone.

According to an aspect of the invention, the device may be a portable device, i.e. a device that can be easily transported to be used on different locations and/or in different rooms of a house. It may be easily carried by one person and transported using for instance a car or a bike. The device may be used in a daycare center, can be taken with the user when visiting relatives or friends and during vacation.

In embodiments a system according to the disclosure can comprise a lifting unit positioned in, on and/or under a mattress, wherein the lifting unit may comprise at least one bladder, a control unit connected to the lifting unit, and a pliable skirt extending from a circumference of the at least one mattress. The control unit can be configured to inflate or deflate the at least one bladder.

In embodiments of a system according to the disclosure a sensor unit can be arranged for detecting change in a body-related parameter of a child or toddler. The sensor unit further can be configured to activate the control unit when detecting the change in the body-related parameter. The lifting unit may provide a rhythmic upward and downward motion to at least an upper body part of a child or toddler on the device, thereby assisting the child or toddler by falling asleep and/or staying asleep.

In embodiments the body-related parameter may be at least one of a respiratory rate of the child, a heart rate of the child, sound produced by the child, position of the child and/or movement of the child and/or a change therein, wherein the system can be arranged for generating a signal based on the sensed body-related parameter and/or change therein. The system can further comprise a processor arranged for receiving said signal, wherein the system may further be arranged for generating an activation signal for the control unit if a predetermined threshold value has been exceeded.

In embodiments in which at least a microphone is provided the system can further be designed such that it can be used as a babyphone. In such system also a camera can be provided for monitoring the child or toddler, especially the baby or toddler or such infant, in a bed, such as but not limited to a children's bed, cod or cradle.

According to an aspect of the invention a mattress may be provided in which a device and/or a system according to the disclosure has been incorporated.

According to another aspect of the disclosure a method for encouraging sleep is provided, in which a support device is used having an at least one plank and a at least one support, wherein the at least one plank can be hingingly connected to the at least one support. A lifting unit is operated for reciprocally tilting the at least one plank relative to the at least one support, preferably at a rate similar to a parent's heart beat or breathing pattern, for example between 5 and 20 times per minute, such as for example between 8 and 15 times per minute, once per second or continuously.

According to another aspect of the disclosure, a method for encouraging sleep is provided, using a device comprising a support device, a control unit and a lifting unit. Said method may comprise the steps of positioning the support device on, in and/or under a mattress, wherein the method further comprises the step of activating the control unit for controlling the lifting unit for alternatingly moving at least part of an upper body part of the child or toddler upward and downward, and/or may comprise the step of deactivating the control unit.

The device in said method can further comprise a sensor unit. The method can further comprise the steps of sensing a body related parameter of a child on the device, and generating a signal based on the sensed body related parameter and/or change therein, receiving and analyzing said signal, generating an activation signal for the control unit if a predetermined threshold value has been exceeded, receiving the signal and / or may comprise the steps of sensing a body related parameter of the child, and generating a signal based on the sensed body related parameter and/or change therein, receiving and analyzing said signal, generating a deactivation signal for the control unit if coming below a predetermined threshold value, and receiving the deactivation signal.

A method according to the disclosure can help a child fall asleep and/or stay asleep by simulating the environment of a womb and/or the soothing sensation of the breathing rhythm of another person, by providing an alternating upward and downward motion of at least part of the body of the child or toddler and/or by generating sounds that help the child or toddler relax. Preferably no involvement of somebody else, such as a carer is needed to start the start and/or stop the device generating the soothing environment. Alternatively, the device may be manually activated and deactivated by a person such as a carer using the device, and/or another person not lying on the device.

In an aspect of the disclosure a computer program product for encouraging sleep is provided not claimed as such, the computer program product comprising computer readable code for causing a processor to perform the step of activating a lifting unit for alternatingly moving at least part of an upper body part of a child or toddler on a mattress upward and downward, and/or the step of deactivating the control unit.

In embodiments the computer program product further performs the steps of receiving a signal based on a sensed body-related parameter and/or change therein, determining whether a predetermined threshold value has been exceeded, generating an activation signal if the predetermined threshold value has been exceeded, receiving the activation signal, receiving a signal based on a sensed body-related parameter and/or change therein, determining whether coming below a predetermined threshold value, generating a deactivation signal if coming below the predetermined threshold value, and receiving the deactivation signal.

In embodiments the computer program product can comprise computer readable code for causing a processor to perform the steps of collecting data associated with the sensed body-related parameter and/or change therein in a database, comparing the data associated with the sensed body-related parameter and/or change therein to the database, determining that the sensed body-related parameter and/or change therein indicates that a child or toddler starts waking up, generating an activation signal, receiving the activation signal, and activating the lifting unit for alternatingly moving at least part of an upper body part of a child or toddler on a mattress upward and downward. A user of the device may indicate, by using for instance a controlling device, which data should be collected and/or how the collected data should be presented to them. Collected data can e.g. give insight in the sleeping pattern of a child or toddler, factors that may positively or negatively influence the quality of sleep and may provide the system itself with the input needed to learn (e.g. by using a neural network or an artificial intelligence) when the child or toddler is likely to wake up. By having insight in the sleeping habits of the child or toddler, the system can learn to predict when a child or toddler starts waking up and thus activate the device to encourage falling (back) asleep and/or staying asleep at this time. Advantageously, the data may be used to provide insight into which aspects of the device have the most influence on the child or toddlers sleep. For example, it may become apparent that a certain frequency of moving up and down helps the child or toddler best, or that a certain sound and/or volume level aids the child or toddler before and/or during sleep. Preferably the software will allow the user to set threshold values for at least some of the data, for example for when a signal as discussed has to be issued.

Further, in embodiments the computer program product may comprise computer readable code for causing a processor to perform the steps of receiving input from a user, generating an activation signal based on the received input, receiving the activation signal, and activating the lifting unit for alternatingly moving at least part of an upper body part of a child or toddler on a mattress upward and downward. Hence, a user can decide to activate and or deactivate the device and/or system. The user may choose to activate e.g. only the lifting unit, only the speaker unit or both e.g. simultaneously or for different periods of time. Preferably the user can provide input to the device on how to function, e.g. with which frequency the tilting motion should alternate, which sound should be provided (a song, white noise, a voice, etc.), the volume with which the sound should be produced and more preferably the user can adjust the sensitivity of the sensors and so on.

A lifting unit for use in a device and/or a system according to the disclosure may comprise a bladder and a control unit, wherein the control unit comprises an air pump connected or connectable to the bladder through at least one tube, wherein the control unit is configured to inflate and/or deflate the bladder according to a programmed rhythm by activating the air pump.

In embodiments of a lifting unit according to the invention, the control unit may comprise the at least one sensor for sensing a body-related parameter or a change in a body-related parameter, and wherein the control unit is further arranged for activating the pump based on a signal received from a sensor sensing a body-related parameter or a change is such body-related parameter. For example a pressure sensor and/or a flow sensor detecting change in pressure and/or movement of fluid in the bladder or a line connecting the bladder to the pump, indicative for movement of a child or toddler using the system. In embodiments the system can be designed such that at all times during use at least some fluid, especially air is present in the bladder and/or the said line or pump, such that movement of a child or toddler using the system can be detected by movement of said fluid, especially said air. By maintaining at least a minimum volume of air and/or a minimum positive pressure inside the at least one bladder during use, the responsiveness of the system can be further improved.

In embodiments, a device and/or system according to the disclosure can be used for waking a child or toddler from sleep, e.g. by generating a sound, wherein the sound can increase in volume over a certain period of time and/or by providing light which may increase in intensity and/or change in color over a certain period of time to simulate a sunrise. Similarly, the device may be used for encouraging a child or toddler to sleep by simulating a sunset, e.g. by slowly dimming light and/or lowering the volume of sound produced.

### BRIEF DESCRIPTION OF THE DRAWINGS

The invention will be further elucidated on the basis of exemplary embodiments which are represented in the drawings. The exemplary embodiments are given by way of non-limitative illustration of the invention. In the drawings:
FIG. 1 shows a schematic perspective view of a device in a bed for encouraging sleep according to the invention;
FIG. 1A schematically shows part of a device of the disclosure, with a bladder deflated;
FIG. 1B in side view schematically a plank and method for defining stiffness of the plank;
FIG. 2 shows a schematic perspective view of a device for encouraging sleep according to the invention;
FIG. 3 shows a schematic drawing of the device according to an aspect of the invention;
FIG. 4 shows a schematic side view of another embodiment according to the invention;
FIG. 5 shows a schematic side view of yet another embodiment according to the invention;
FIG. 6 shows a schematic side view of an embodiment according to the invention;
FIG. 7A shows a schematic side view of an alternative embodiment according to the invention in a first position;
FIG. 7B shows a schematic side view of the alternative embodiment of FIG. 7A in a second position;
FIG. 8 shows a schematic side view of an embodiment of a device of the disclosure;
FIG. 9 shows schematically in isometric view part of a further embodiment of a device of the disclosure.
FIG. 10 shows schematically exploded view of another embodiment according to the invention;
FIG. 11A shows schematically an cross-sectional isometric view, in which the bladders are deflated;
FIG. 11B shows schematically an cross-sectional isometric view, in which the bladders are inflated;
FIG. 11C shows a schematic front view of the support device, bladders and cover of an embodiment according to the invention;
FIG. 12 shows schematically an embodiment of a pump of the pump system;
FIG 13 A and B show schematically a pump system or part thereof, with a pump chamber fully expanded (fig. 13A) and with pre-pressure (fig. 13B); and
FIG. 14 in cross sectional view schematically a device according to the disclosure in a further advantageous embodiment.

### DETAILED DESCRIPTION

In embodiments the invention relates to a device for a cot or child's bed to encourage sleep, comprising, a support device to be placed in or under a mattress, comprising at least one plank and at least one support. The device further comprises a lifting unit, wherein the lifting unit comprises at least one bladder, preferably at least two bladders, more preferably four bladders, positioned between the at least one plank and the at least one support. The lifting unit further comprises a control unit, wherein the control unit is configured to inflate and/or deflate, preferably actively inflate and actively deflate the at least one bladder via a pump of a pump system for providing an alternating upward and downward motion, which can be a tilting movement, of the at least one plank. In addition, the device further comprises a pliable skirt extending from a circumference of the at least one plank.

In this description encouraging sleep should be understood as at least meaning aiding the toddler or child in falling asleep or falling back asleep, staying asleep and/or relaxing, such as for example but not limited to stop crying.

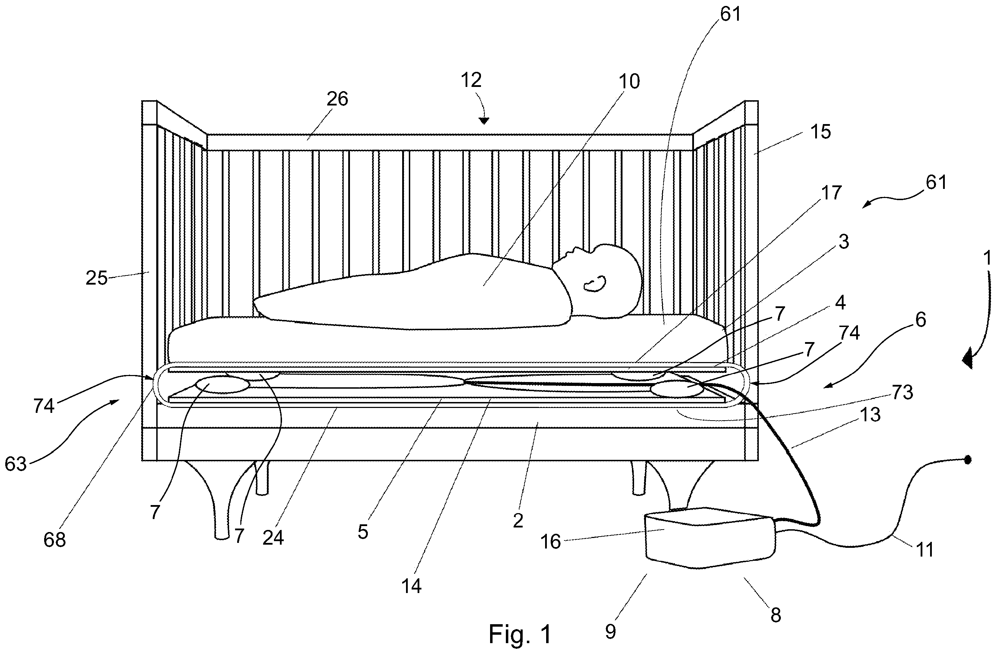

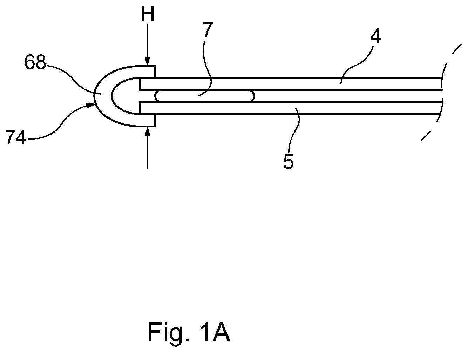

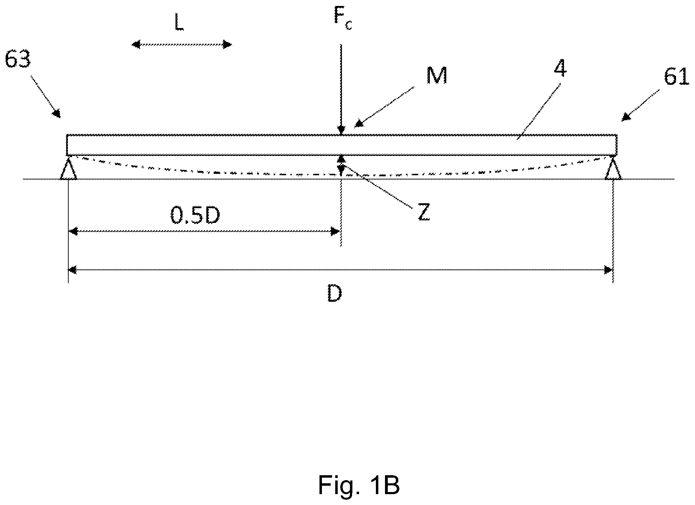

FIG. 1 shows a schematic perspective view of an embodiment of a device 1 for encouraging sleep according to the invention. Encouraging sleep can in this description for example be understood as stimulating a baby or toddler, to fall asleep, or to fall back asleep, or to prevent a baby or toddler from waking up, or preventing a baby or toddler from crying when waking up, for example before falling asleep again. The device 1 comprises a support device 2 to be placed in, on or under a mattress 3, comprising at least one plank 4, and a at least one support 5.

In this description a plank should be understood as a relatively stiff element or combination of elements, on which a mattress can rest during use, and with which said mattress can at least partly be lifted. A plank can for example be but is not limited to a hollow or solid element or a layered element, or for example a frame with slats, and can for example be made of wood, metal or plastic, or a combination thereof. A plank 4 is preferably provided such that when the weight of a baby or toddler is resting on the plank or on a mattress provided on the plank the plank will not deform to such an extend when a head end 61 or feet end 63 of the plank 4 is pushed upward that the child or toddler is not lifted by the plank. Preferably the child or toddler in such case is lifted over a distance Z which is at least 25% of the distance over which said head end 61 or feet end 63 is lifted. Whereas the plank should preferably not deform to such extend that the baby or toddler will roll due to said movement.

Stiff or at least relatively stiff in this disclosure can be understood as meaning that if the plank 4 is supported at two opposite longitudinal ends thereof, for example at a head end 61 and a feet end 63, as schematically shown in fig. 1B, in a horizontal position, and a vertical force F_{c} is exerted to the middle M of the plank 4, i.e. midway between the head end 61 and the feet end 63, which force F_{c} is 150 N, the middle M of the plank 4 will be pushed down over a distance Z which is relatively small, for example less than 1/10^{th} (10%) of the distance D between feet end 63 and head end 61. In advantageous embodiments the distance Z will be no more than 8%, such as no more than 6%, such as for example no more than 4% of the distance D between the said feet end 63 and the said head end 61, preferably no more than 3%, more preferably no more than 2% such as for example between 0 and 1.5% of said distance. For a plank 4 having for example a length of 1.4 meter, i.e. a distance D between the head end 61 and the feet end 63 of the plank 4 of 140 cm, this means that for Z=4% or less, the middle M of the plank 4 will not be pushed down over a distance Z of more than 5 cm (0.05 meter), preferably not more than 4 cm, more preferably nor more than 3 cm, such as for example between 0 and 2 cm. Preferably the plank 4 is similarly stiff in a direction perpendicular to the longitudinal direction L of the plank 4 between head end 61 and feet end 63. It will be clear that a plank 4 can also have a different length L, such as for example 1.2 meters, or between 40 and 100 cm, such as for example between 60 and 90 cm, such as for example 80 cm. The shorter the distance D or the length L, the larger the relative distance Z in % can be allowed to be, although a stiffer plank is preferred.

The support 5 can be stiff, similar to the plank 4, but it has been found that the support 5 can also be made much less stiff, such as flexible or at least more flexible than the plank 4, especially when the device is used in a bed providing support for bladders of the device near the head end 61 and the feet end 63.

The mattress 3 shown in FIG.1 is located in a bed 12, wherein the bed comprises a bottom portion 24, a headboard 15, a footboard 25 and a side portion 26. In this disclosure a bed should be understood as meaning a children's bed, a cod or a cradle. The bottom portion 24 can be a bed bottom as known in the art and/or can be formed by or comprise the at least one support 5 of the device 1, as will be discussed.

The device 1 further comprises a lifting unit 6. The lifting unit 6 in this embodiment comprises at least one bladder 7, which is positioned between the at least one plank 4 and the at least one support 5. The bladder 7 is a flexible expansion body that, when deflated, can be relatively flat to ensure that in a deactivated state the child or toddler 10 lying on the mattress 3 does not feel the presence of device 1. Preferably the height of the bladder 7 and at least one plank 4 in deactivated state is relatively small, for example compared to a height of the bed and/or the mattress, and can for example be less than 3 cm, e.g. less than 2 cm, such as less than 1 cm. More preferably the height H of the bladder 7, at least one plank 4 and at least one support 5, in deactivated state as for example shown in fig. 1A, is less than 3 cm, e.g. less than 2 cm. In embodiments a single bladder 7 can be positioned at a longitudinal center line of the support device between near a head end 61 of the bed 12. In embodiments the device 1 can be provided with multiple bladders 7 having either the same and/or varying sizes. Preferably during use the plank 4 is stiff to such extend that the plank 4 will not touch the support 5 during use. Preferably a plank 4 of a device 1 of the disclosure is stiff, such that it substantially does not deform during use. In embodiments, as will be discussed, the device comprises a cover 75, wherein part of the cover extends over the plank and comprises part of the cover elastically shaped over a peripheral edge of at least the plank, such that the said part of the layer biases the cover outward, forming the skirt.

FIG. 1 shows four bladders 7, two bladders 7 near a head end 61 of the bed 12 and two bladders 7 near the foot end 63 of the bed 12. In embodiments the at least one plank 4 can be a rectangular sheet, made out of wood, plastic or a metal. The at least one plank can also comprise holes or open spaces and can be made out of multiple different components for example as commonly used in a slatted base. The support 5 can be a single support, such as a further plank or the bed bottom, or can comprise multiple supports or support elements, such as one for each bladder or for each set of bladders. Furthermore, in embodiments a skirt 68 is provided, extending from the circumference of the at least one plank 4. The skirt will be discussed further later in the figure description. By using two sets of bladders 7, one set at a head end 61 and one set at a foot end 63 of the bed 12, supporting the plank 4, the plank 4 can tilt and can translate up and down, or combinations thereof. In such embodiments the plank 4 need not and preferably is not connected to the at least one support 5 by a physical hinge or the like. In the embodiments shown the plank 4 and support 5 can be held together by for example the skirt 68 or a cover 74 or the like.

The lifting unit 6 also comprises a control unit 8, wherein the control unit 8 is configured to inflate and/or deflate the or each bladder 7 for providing an alternating upward and downward or tilting motion. In embodiments the control unit 8 can be configured to alternatingly inflate and deflate the bladder or bladders 7 in a rhythm simulating the breathing rhythm of an adult person. The control unit 8 can comprise a pump system P, for forcing air through flexible tube 13 to bladder 7 in order to inflate the bladder 7, thereby pushing plank 4, which may also be referred to as for example upper panel or board 4, and correspondingly the child or toddler 10 lying on the mattress 3 upwards. By providing the air in a pulsated manner, bladder 7 gets time to deflate due to pressure exerted on the bladder through the gravity force working on the child or toddler 10 lying on the mattress, pushing the upper panel 4 down towards the support 5, which may also be referred to as lower panel of board 5. In embodiments, the pump system P preferably both inflates and deflates bladder 7 to provide the upward and downward motion respectively.

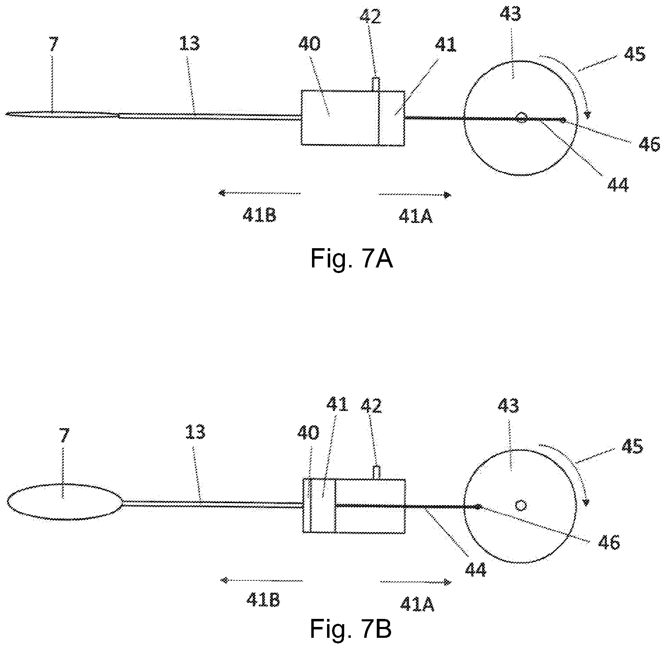

In fig. 7A and B an embodiment is shown in which the bladder 7 is connected to a pump chamber 40, which has a movable and/or deformable wall part 41 that can be moved for reducing and increasing the volume of the pump chamber 40. An air inlet 42, preferably comprising a one way valve or a valve operable by the control unit 8 is provided for allowing air into the pump chamber 40 when desired. In the embodiment shown a movable wall part 41 is shown by way of a piston movable in a cylinder. The piston 41 is drivable by a drive wheel 43, connected to the piston by a drive shaft 44. By rotating the wheel 43 for example clockwise, as indicated by arrow 45, the piston 41 will be moved reciprocally forward and backward, between the most rearward position as shown in fig. 7A and the most forward position as shown in fig. 7B. as is known in the art, by moving the connecting point 46 of the drive shaft 44 to the wheel 43 the stroke of the piston 41 can be changed. In this embodiment the inlet 42 is shown positioned such that if the piston 41 is moved backward, the inlet 42 is available for letting air into the chamber 40, whereas when it is moved forward the inlet 42 is blocked by the piston 41 or does not open into the chamber 40. Obviously other means for driving the wall part 41 can be used, as known to the skilled person.

Such embodiment can be used as follows. At the start of use the wall part 41 can be moved backward, in direction 41A, such that the volume of the chamber 40 is increased, preferably maximized. Air is sucked into the chamber 40, from the bladder 7 and/or from the surroundings S through the inlet 42. Then the wall 41 is moved forward, in direction 41B, reducing the volume of the chamber 40 and pushing the air into the bladder 7, inflating the bladder 7. After a period of time the wall 41 is moved backward again, in direction 41A, increasing the volume of the chamber 40 again and sucking air from the bladder 7. In such back stroke of the wall 41 preferably the inlet 42 is closed, such that no air is introduced into the chamber other than from the bladder 7. It will be understood that by moving the wall 41 back and forth in this manner in a reciprocal manner, the bladder will be inflated and deflated reciprocally. During such use the pressure inside the system can be monitored and the amount of air in the system can be controlled based on such pressure. This allows for example to increase the pressure by adding additional air when a child or toddler is detected to be lying more to a side of the at least one plank, or lower the pressure by removing some of the air from the system if the child or toddler has moved partly away from the at least one plank 4. Moreover such embodiment has the advantage that any leaks in the system can be compensated for by the air inlet 42. Alternatively the chamber 40 with the wall part 41 can be used directly for moving the at least one plank 4 hinging relative to the at least one support.

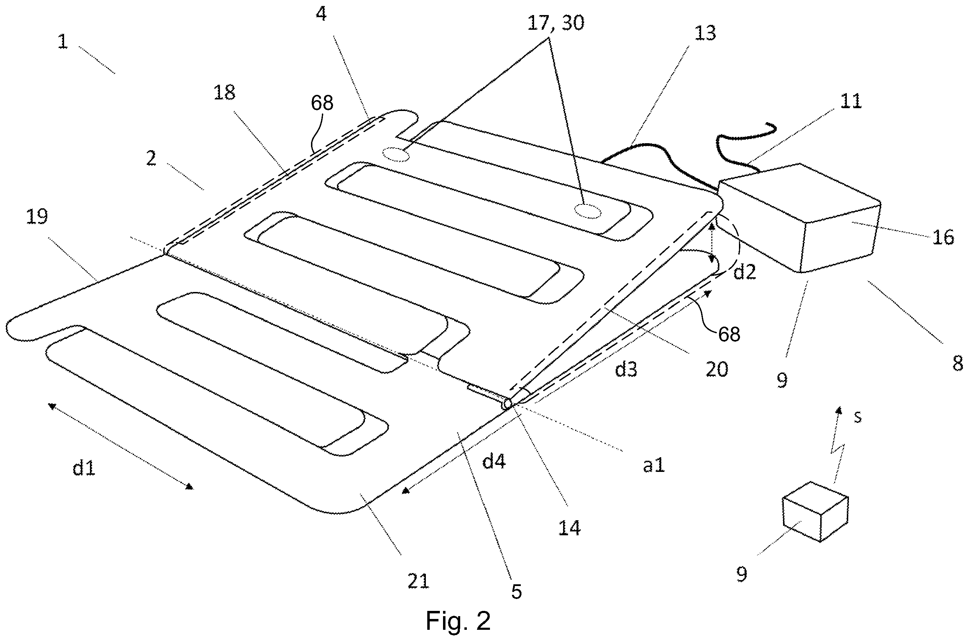

In the alternative embodiment shown in FIG. 2, the at least one plank 4 is further connected to the at least one support 5 through the hinge 14. The hinge allows the upper panel or board 4 to pivot relative to the support 5, for example when inflating or deflating a bladder 7 or activating a different lifting unit 6. The hinge 14 can be any suitable connection arranged for providing a pivoting movement of the at least one plank 4 relative to the lower panel 5, preferably without movement of the at least one plank in a longitudinal direction along the lower panel 5. The hinge 14 can for example be formed as a pin-hole hinge, another type of hinge, either integrated in the support device 2 or a separate connection piece, and/or a piece of fabric, rubber or any supple and/or flexible material connecting the at least one plank 4 to at least one support 5. In the embodiment shown in FIG. 2, the at least one plank 4 is hinging upward relative to the support 5, which remains stationary.

In the embodiment shown in FIG. 1 the lower panel 5 is supported on the bottom portion 24 of the bed 12. The lower panel 5 may also be supported on or in the mattress, or on another surface on which a mattress normally may be placed, such as a floor.

The device 1 in the embodiment of FIG. 1 further comprises a sensor unit 9 arranged for detecting at least one body-related parameter or a change in at least one body-related parameter of a child or toddler 10, wherein the sensor unit 9 is configured to issue a control signal S upon detecting for example a relevant change in said at least one body related parameter, for example a change such that the body related parameter exceeds a predefined threshold value or a change above or below a threshold value. The control unit 8 may comprise a receiver 8A for receiving the control signal S. The control signal S can then activate the control unit 8, for example starting or modulating the pump 64 for periodically, preferably rhythmically inflating and deflating the bladder 7, as described. Alternatively the sensor unit 9 could be omitted, the system activatable for example by a user through a switch or timer.

As shown in the embodiment shown in FIG. 1, the device 1 can be powered by an external power source through power cord 11, for example through a mains socket. However, the device 1 can also be powered using for instance batteries, solar power, or other known power sources.

The device 1 can further comprise a speaker unit 16, as shown in fig. 1. This speaker unit 16 can be part of the control unit 8, but might also be a separate device communicatively connected to the control unit 8 and/or a different controlling device such as a computer, tablet or smartphone, using a wired or wireless connection (e.g. Bluetooth or Wi-Fi). The speaker unit 16 can be powered by the control unit 8 and/or comprise its own power source such as batteries or a solar power element.

FIG. 2 shows a schematic perspective view of a possible embodiment of the device 1 of the disclosure, wherein the sensor unit 9 comprises at least one sensor element 17 for sensing at least one of a respiratory rate of the child or toddler, a heart rate of the child or toddler, sound produced by the child or toddler, position of the child or toddler and/or movement of the child or toddler and/or changes therein. The sensor element 17 in the embodiment shown in FIG. 2, is located on the upper panel 4. The sensor element 17 can however also be positioned differently, for example but not limited to in and/or on the bladder 7, between the upper panel 4 and support 5 or under the support 5. Moreover, the sensor element 17 can also be a separate element placed close to the child or toddler 10, for example releasable connected to the bed 12 using suction cups, Velcro, a support device such as a hook to be placed over a railing of bed 12 or a more permanent connection using fastening means such as screws, nails, snaps, etc. Additionally, it might be lying loose on the mattress or placed further from the child or toddler, such as outside of the bed 12. The sensor element can for instance be integrated in control unit 8.

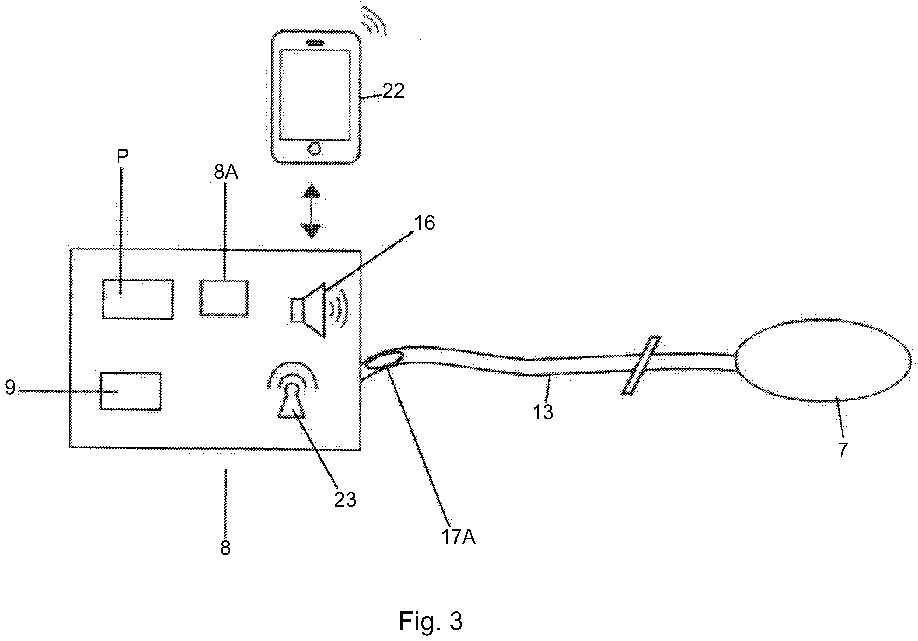

FIG. 3 shows a schematic drawing of the device 1 according to an aspect of the invention. The sensor element 17 can be communicatively connected to the control, for instance through antenna 23, unit 8 and/or have a communicative connection with another controlling device 22, such as a computer, tablet or smartphone, using a wired or wireless connection (e.g. Bluetooth or Wi-Fi). Optionally, the sensor 17 can be a sensor in close contact with the body of the child or toddler 10, such as a wearable sensor (e.g. a sensor wore on the wrist or across the chest). For sensing the body-related parameter of the child or toddler 10 a single sensor element 17 may be used, however the device 1 can also comprise a plurality of sensor elements 17 forming a sensor network communicatively connected to each other and/or connected to a controlling device 22. Each sensor element 17 may sense one body-related parameter, such as only the respiratory rate or only the heart rate, but the sensor element 17 can also be configured to sense multiple body-related parameters for example both respiratory rate and heartbeat rate. The sensor element 17 may among others be or comprise a sound sensor, an occupancy sensor, pressure sensor, flow sensor, optical sensor, movement sensor, heartbeat sensor or a light sensor. By integrating a pressure sensor 17A and/or a flow sensor 17A in the system, especially for sensing air pressure and/or air flow directly or indirectly in the bladder 7 or a line between the bladder and the pump 64, it can easily be detected whether a child or toddler is lying on the system and/or is moving on the system, especially if at least some air is kept in the bladder and/or said line and pump.

In embodiments the sensor element 17 can be or comprise a microphone 30, with which sounds from the child or toddler 10 can be registered, for example crying or talking of a baby or toddler. In the control unit 8 a threshold value can be set, wherein the control unit 8 can be programmed to start the pump unit P when the sound level produced by the child or toddler 10 exceeds the threshold value. Thus the lifting device 6 can be started automatically when for example a baby starts crying. Preferably the control unit 8 can distinguish between different types of sounds, which may prevent the control unit 8 to activate the pump unit P and/or the speaker 16 unintentionally, e.g. when carers such as parents enter a room to check up on the baby or toddler, or when the baby or toddler is contently babbling, instead of crying. In embodiments the sensor element 17 can be or comprise a movement sensor. Similarly, in the control unit 8 a threshold value can also be set for other parameters, such as movement of the child or toddler, wherein the control unit 8 can be programmed to start the pump unit P when the movement level produced by the child or toddler exceeds the threshold value, or when a predefined pattern of movement or change in movement or change in movement patterns is detected. Thus the lifting device 6 can be started automatically when for example a baby starts moving around in a manner indicative for a baby to start waking up or feeling uncomfortable, but may not activate unintentionally when a carer checks up on the child or toddler 10. The lifting device 6 may be controlled automatically, as described herein, and/or the lifting device 6 can be controlled by the child or toddler 10 or a person other than the child or toddler 10 lying on the mattress 3, for instance a cares such as a parent of a toddler trying to sleep. The lifting device 6 may be activated using the control unit 8 and/or by activating the device 1 using the controlling device 22. Optionally, the device 1 may be activated using a timer. If for instance the child or toddler 10 is prone to waking up at a specific time, the timer can be set to activate the device 1 at said specific time or shortly prior to said time to prevent the child or toddler waking up.

Embodiments comprising a microphone 30 can advantageously also be used as a babyphone, even if for example the system is not used for moving the at least one plank up and down. Also a camera may be provided for monitoring the child or toddler in the bed 12.

In a system using a microphone 30 preferably the control unit comprises an algorithm for recognizing a predestined type of noise, and especially for ignoring other noises. For example such algorithm can be designed to recognize a child or toddlers crying as indicative for the necessity to react, whereas other types of noises, such as for example the baby or toddler babbling contently, people walking, traffic, falling objects and the like may be ignored by the system.

Moreover, the controlling device 22 and/or the control unit 8 may be configured to collect and process data about the body-related parameters to provide insight into for instance the sleeping habits of the child or toddler 10, quality of sleep, nightly recharge (i.e. did the body recover and/or the autonomic nervous system recover). Additionally, the controlling device 22 and/or control unit 8 may comprise a neural network, which can be used to train the device 22 and/or control unit 8 via the collected body-related parameter data. The device 1 can then for instance predict when the child or toddler 10 is prone to waking up and activate at a specific time or when a change is detected in certain body-related parameters before the threshold is exceeded.

After activation, the device 1 can be configured to deactivate after a predetermined time period after activation. This may for instance be a time period after initial activation or after the last detected moment when the threshold value of the body-related parameter was exceeded, i.e. a time period after the child or toddler 10 has fallen back asleep. Additionally, the device 1 may be activated and/or deactivated manually, using the control unit 8 and/or the controlling device 22, for example using an app.

Further, the device 1 can be configured to activate when a change in the body-related parameter exceeds a predefined threshold value and configured to deactivate when the change in the body-related parameters comes below a predefined threshold value, which threshold values may be the same or different.

In embodiments the speaker unit 16 is configured for generating a sound, for example a voice of a parent, white noise, a heartbeat or a song or another comforting sound, for a predetermined time period upon activation of the control unit 8 by the sensor unit 9. A heartbeat can for example be a prerecorded heartbeat, for example a heartbeat of a parent or of another carer. As described herein, the speaker unit 16 may be integrated in the control unit 8, or be a separate device. Hence, the speaker unit may be automatically activated upon activation of the lifting unit 6 and may be active during the same time period the lifting unit 6 is active. However, the speaker unit may also be active for another duration, e.g. a longer or shorter time period than the time period the lifting unit 6 is active. Additionally, the speaker unit may be activated and deactivated manually, using the control unit 8, the controlling device 22 and/or using a activation and deactivation on the speaker unit 16 itself. Optionally, a person, e.g. a parent, can speak into the controlling device 22 for a preferred period of time.

FIG. 2 further shows that the support device 2, especially the at least one plank 4 and the at least one support 5, can comprise left portions 18, 19 and right portions 20, 21, wherein the left portions 18, 19 and the right portions 20, 21 are adjustable relative to each other in a direction d1 perpendicular to the direction d2 in which the at least one plank 4 moves relative to the at least one support 5 for adaption to a width of the mattress 3. Thus, by adjusting the left portions 18, 19 and right portions 18, 19 relative to each other, the support device 2 can be made to fit different sizes of beds, thereby enabling the upward and downward motion of at least part of an upper body part of the child or toddler, e.g. an upper torso and head of the child or toddler 10 lying on the mattress 3 regardless of whether the child or toddler lies in the center of the mattress 3 or close to either the left or right side of the mattress, i.e. close to the side portion 26. In addition, due to the tight fit of the support device 2 with the bed, a safer device 1 is created, since it may be prevented that the child or toddler 10 becomes trapped between a portion of the mattress that moved upward and a side portion 26 of the bed 12 or, in case the bed does not have high side portions 26 or a wall next to the bed, run the risk of rolling out of the bed due to a slope created by a too narrow support device 2 moving upward. The at least one plank 4 can be of the same width as the at least one support 5, or in embodiments the at least one plank 4 can be smaller or wider than the at least one support 5 or vice versa.

Adjusting the width may be realized by sliding the left parts 18, 19 and right parts 20, 21 relative to each other by providing for instance a protrusion and groove structure, wherein the parts 18,19, 20, 21 can slide continuously using a frictional force between the left part 18, 19 and right part 20, 21 to enable that the chosen width is maintained during use, and/or wherein predetermined positions are provided to lock the left part 18, 19 and right part 18, 19 during use. Preferably, the sliding system comprises a locking system preventing the left and right parts to slide too far away from each other and/or to prevent the parts from becoming separated. However, in another embodiment the support device 2 might comprise a separate left part 18, 19 and separate right part 20, 21, wherein the two parts can be connected by for instance a snap connection, wherein the snap connection can be realized at predetermined positions to obtain the desired width of support device 2.

Alternatively a single piece upper panel 4 can be used in combination with a single piece lower panel 5, wherein the hinge element 14 and the lifting unit 7 can be adjusted such that a side portion of the support device can be removed (e.g. by sawing off) to obtain the desired width. Optionally, different predetermined sizes of the support device may be provided to fit different bed sizes.

In embodiments a pressure sensor may be mounted in the tube 13 and/or the control unit 8 for sensing pressure changes in the bladder 7 by detecting a counter pressure. When the child or toddler 10 lies on mattress 3, the pressure sensor may detect for instance a position of the child or toddler 10 on the mattress 3 or movements made by the child or toddler 10. Optionally, when a small volume of air is present in the bladder 7 when the device 1 is deactivated, the pressure sensor may detect when the child or toddler 10 is lying down on the mattress 3 and the device 1 can for instance come out of an energy saving state.

The at least one plank 4 can be hingeably connected to the at least one support 5. The device 1 shown in FIG. 2 has upper panel 4 that is connected to the support 5 by the hinge 14, wherein the hinge 14 is positioned at substantially the longitudinal middle of the at least one support 5. However, the hinge 14 may also be positioned differently, for example closer towards the middle, or further away from the middle of at least one support 5. Additionally, the plank 4 has the same width as the support 5, but a smaller surface area. By inflating the bladder 7, the plank 4 moves upward in the direction d2, hinging around a hinging axis a1. The child or toddler 10 lying on the mattress 3 will then experience the movement of the mattress 3 top surface upwardly.

The at least one plank 4 is arranged for hinging upward about a hinging axis a1 and the at least one support 5 comprises a first portion extending from the hinging axis a1 in a first direction d3 that is perpendicular to the hinging axis a1 to below the at least one plank, and a second portion extending from the hinging axis a1 in a direction d4 opposite to the first direction d3. The hinge 14 is positioned such that the lower panel 5 extends beyond the upper panel 4, thereby creating additional support towards the bottom portion 24 of the bed 12 and thus creating extra stability of the support device 2 in case the child or toddler 10 does not lie along the longitudinal center line of the support device 2, but towards a side portion 26 of the bed 12. Additionally, by having one hinging element 14 and/or multiple hinging elements 14 covering substantially the entire width (in direction d1) of the support device 2, the pressure exerted on the support device 2 is evenly divided regardless of the position of the child or toddler 10 lying on mattress 3. Further, to ensure the mattress 3 moves upward over the surface area of the upper panel 4, and not only at the position of the tilting unit, such as the bladder 7, the upper panel 4 is relatively stiff. The upper panel 4 preferably is stiffer than the mattress 3 and such that it only deforms minimally under the pressure exerted on the panel 4 by the child or toddler 10. Optionally, the upper panel 4 may deform, but only such that the child or toddler 10 is still moved upward, regardless of the position on the mattress 3 without being in danger of being trapped between a side wall of the bed and the mattress 3. The different portions of panels 4 and 5 of support device 2 may comprise holes and/or may be of one piece.

The hinge 14 can be an actual hinge or can be a virtual hinge, for example when the at least one plank 4 and the at least one support 5 are not physically connected to each other for hinging, but are for example connected to each other by the bladders 7 and the skirt 68 or a cover 73 enclosing the device 1, allowing not only tilting of the plank 4 relative to the at least one support 5 but also an up- and down translation, of combinations thereof.

By adjusting the position of the bladder(s) 7 longitudinally, for example towards or away from the position of the hinge 14, a lower or higher upward movement of the upper panel 4 may be achieved. The bladder 7 may be relatively small, i.e. have a relatively small volume, e.g. less than 1 liter, such as less than 0,5 liter, so that a relatively small pump may be used to create sufficient upward and downward movement to simulate the breathing rhythm of a child or toddler and a short response time can be realized. In embodiments the volume can for example be around 200 ml. Alternatively instead of or in addition to a bladder a different device can be used for reciprocally moving, i.e. tilting of the at least one plank, such as a mechanical drive, an electro-mechanical drive or a magnetic drive, as known in the art.

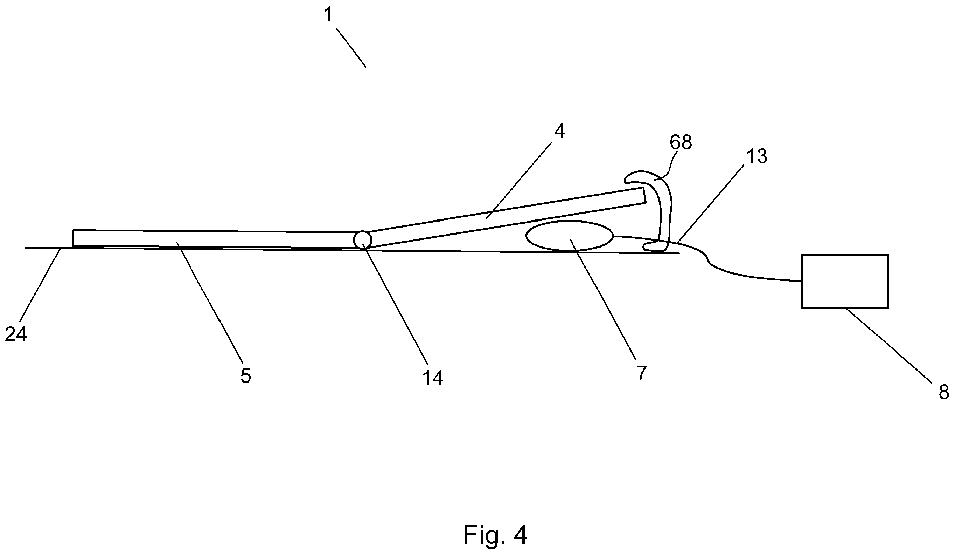

FIG. 4 shows a schematic side view of another embodiment according to the invention. In the embodiment of FIG. 4, the support 5 is positioned in front of the upper panel 4, but does not extend beyond hinge 14 underneath plank 4. Hence, the bladder 7 or other tilting unit is positioned between the bottom portion 24 of the bed 12 and the upper panel 4. The bladder 7 pushes the upper panel 4 upward when inflated, due to the support (counterforce) given by the bottom portion 24 of the bed 12.

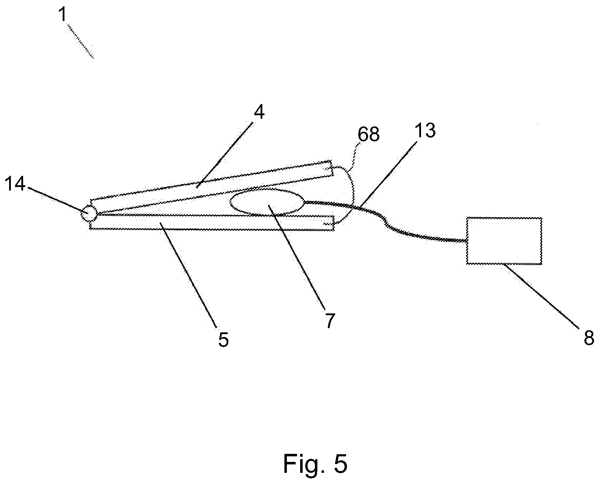

FIG. 5 shows a schematic side view of yet another embodiment according to the invention in which the support 5 is positioned under plank 4, but does not extend beyond hinge 14. In an embodiment the lower panel 5 may be attached to a surface on which the lower panel 5 is placed, such as the bottom portion of the bed 24, using for instance straps, Velcro or fastening means such as screws, nails or an adhesive.

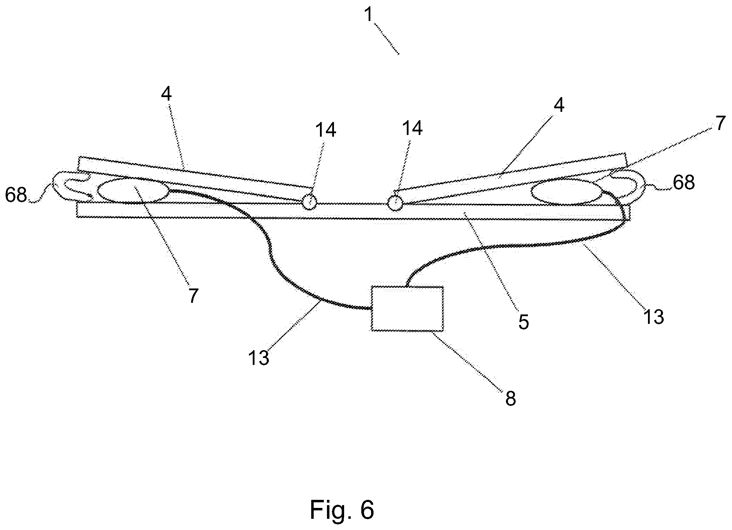

FIG. 6 shows a schematic side view of an embodiment according to the invention in which the lifting unit 6 might be located at the end towards headboard 15, as well as towards footboard 25. By having lifting units 6 on both ends of the bed, including an additional hinge 14 and additional upper panel 4, the device 1 can compensate for when the child or toddler 10 lies with his/her head towards the footboard 25, instead of towards the headboard 15. A pressure sensor may be used to sense how the child or toddler 10 is positioned in the bed, i.e. with his/her head close to the headboard 15 or with his/her head close to the footboard 25.

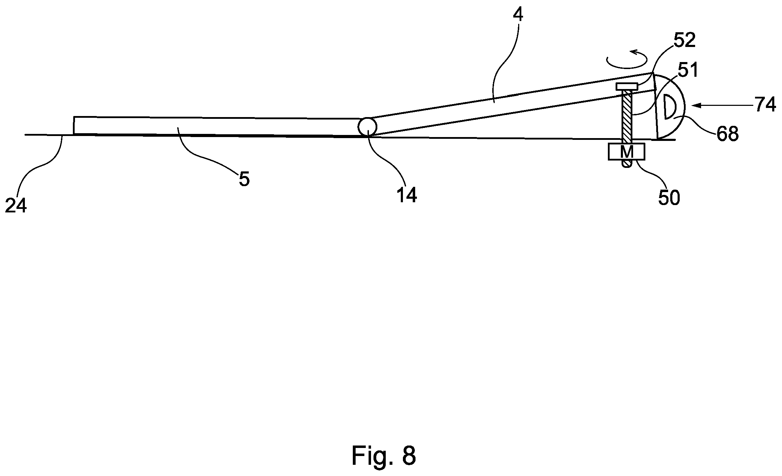

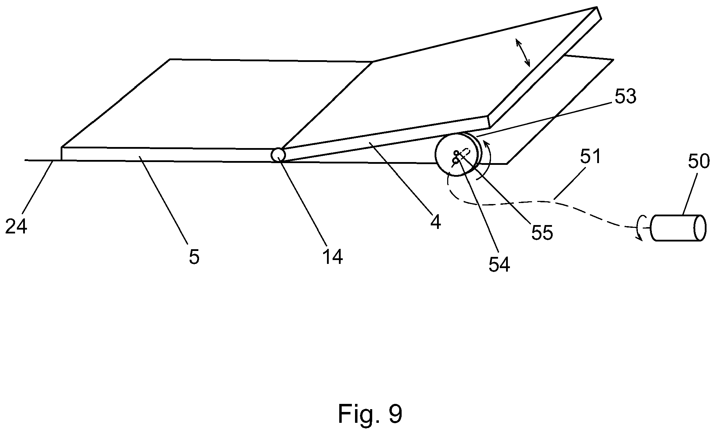

Fig. 8 and 9 show alternative embodiments of a device 1, wherein instead of a bladder 7 an alternative lifting unit 6 is provided for obtaining the tilting movement of the upper panel 4.

In the embodiment of fig. 8 a motor 50 is provided, connected to a spindle 51 with outer screw threads, which spindle 51 can be rotated by the motor 50 around a longitudinal axis of the spindle 51. A nut 52 or other connecting element is mounted on the spindle 51 and secured to the upper panel 4, such that by rotating the spindle 51 the spindle is moved in an upward or downward direction, relative to the motor 50, thereby pushing the upper panel 4 upward or pulling it downward, for obtaining the tilting movement. The motor 50 can be provided directly under the movable part 4, or the motor 50 can comprise a drive element connected to the spindle 51, drivable by a motor element connected to the drive element by a drive axle, for example a flexible drive axle, such that the motor element can be positioned at a distance from the panels, especially from the upper panel 4, for example outside the bed with which the device is used.

In the embodiment of fig. 9 again a motor 50 is provided, with an axle such as a flexible axle 51. A wheel 53 is connected to the axle 51, which wheel 53 is supported by an axis 54 extending substantially parallel to the hinge 14. The axis 54 is connected to or forms part of the axle 51 and is positioned spaced apart from a center 55 of the wheel 53, such that by rotation of the axis 54 the center 55 of the wheel 54 and hence the outer surface 56 engaging the upper panel 4 will move in an up and down movement. The wheel 53, acting as a cam plate engages the lower side of the upper panel 4, the cam plate having an outer peripheral surface which is not rotational symmetrical relative to the axis 54, such that by engaging the lower side of the panel by said outer peripheral surface and rotating the wheel 53, the upper panel will be forced into the reciprocal tilting movement as desired.

In embodiments the axle can be moved reciprocal, alternatingly in a clockwise and an anti-clockwise direction, for obtaining the desired reciprocal tilting movement of the upper panel 4. Alternatively the axle can be rotated constantly in the same clockwise or anti-clockwise direction, rotating the wheel 53 below the upper panel.

In the embodiment shown in fig. 9 the motor 50 is positioned spaced apart from the wheel, connected thereto by a flexible drive shaft or axle 51. Thus the motor 50 can be placed outside the bed or at least outside the reach of the person using the device. Alternatively the motor 50 can be connected directly to the wheel through the axis 54.

The device 1 is preferably a portable device, making it possible to place the device 1 in another bed 12. The device 1 can for instance be taken to a daycare center for a toddler to take his/her daily nap or on holiday for encouraging sleep in a hotel, holiday apartment or mobile home especially when the support device 2 is an adjustable element and can be made to fit different beds. In other embodiments, the device 1 does not comprise the support device 2, making it even smaller during transport.

In embodiments the invention may relate to a system for encouraging sleep, comprising, a lifting unit 6 positioned in, on or under a mattress 3, wherein the lifting unit 6 may comprise a bladder 7. The system may further comprise a control unit 8 connected to the lifting unit 6, wherein the control unit 8 is configured to operate the lifting unit 6, such as for example inflate or deflate a bladder 7, and may comprise a sensor unit 9 arranged for detecting change in a body-related parameter of a child or toddler 10, wherein the sensor unit 9 further is configured to activate the control unit 8 when detecting the change in the body-related parameter. The body-related parameter can be at least one of the respiratory rate of the child or toddler 10, the heart rate of the child or toddler 10, sound produced by the child or toddler 10, position of the child or toddler 10 and/or movement of the child or toddler 10 and/or a change therein. The system can further be arranged for generating a signal based on the sensed body-related parameter and/or change therein, and comprises a processor arranged for receiving said signal and generating an activation signal for the control unit 8 if a predetermined threshold value has been exceeded.

In embodiments the body-related parameter may be at least one of a respiratory rate of the child or toddler 10, a heart rate of the child or toddler 10, sound produced by the child or toddler 10, position of the child or toddler and/or movement of the child or toddler and/or a change therein, wherein the system can be arranged for generating a signal based on the sensed body-related parameter and/or change therein. The system can further comprise a processor arranged for receiving said signal and wherein the system may further be arranged for generating an activation signal for the control unit 8 if a predetermined threshold value has been exceeded.

In embodiments the system can alternative to or additional to the sensor unit 9 comprise other devices to switch on and/or off the system, or at least the reciprocal movement of the lifting unit 6, for example a manual or electronic switch or a timer.

In embodiments, a mattress 3 may be provided in which the device 1 and/or system for encouraging sleep has been incorporated.

According to another aspect of the invention, a method for encouraging sleep is provided, using a device 1 comprising a support device 2, a control unit 8 and a lifting unit 6, said method comprising the steps of positioning the support device 2 on, in and/or under a mattress 3. The method may further use a sensor unit 9. The method may further comprise the steps of sensing a body-related parameter of a child or toddler 10 on the device 1, and generating a signal based on the sensed body-related parameter and/or change therein, receiving and analyzing said signal, generating an activation signal for the control unit 8 if a predetermined threshold value has been exceeded, receiving the activation signal, and activating the control unit 8 for controlling the lifting unit 6 for alternatingly moving at least part of an upper body part of the child or toddler 10 upward and downward. and/or the steps of sensing a body-related parameter of the child or toddler 10, and generating a signal based on the sensed body-related parameter and/or change therein, receiving and analyzing said signal, generating a deactivation signal for the control unit 8 if coming below a predetermined threshold value, receiving the deactivation signal, and deactivating the control unit 8.

In embodiments a computer program product for encouraging sleep is provided not claimed as such, the computer program product comprising computer readable code for causing a processor to perform the steps of activating a lifting unit 6 for alternatingly moving at least part of an upper body part of a child or toddler 10 on a mattress 3 upward and downward, and may further cause the steps of receiving a signal based on a sensed body-related parameter and/or change therein, determining whether a predetermined threshold value has been exceeded, generating an activation signal if the predetermined threshold value has been exceeded, receiving the activation signal. Further, a computer program product may also comprise causing the processor to perform the steps of, receiving a signal based on a sensed body-related parameter and/or change therein, determining whether coming below a predetermined threshold value, generating a deactivation signal if coming below the predetermined threshold value, receiving the deactivation signal, and deactivating the control unit 8.

In embodiments a computer program product can comprise computer readable code for causing a processor to perform the steps of collecting data associated with the sensed body-related parameter and/or change therein in a database, comparing the data associated with the sensed body-related parameter and/or change therein to the database, determining that the sensed body-related parameter and/or change therein indicates that a child or toddler starts waking up, generating an activation signal, receiving the activation signal, and activating the lifting unit 6 for alternatingly moving at least part of an upper body part of a child or toddler 10 on a mattress 3 upward and downward.

Further, in embodiments a computer program product may comprise computer readable code for causing a processor to perform the steps of receiving input from a user, generating an activation signal based on the received input, receiving the activation signal, and activating the lifting unit 6 for alternatingly moving at least part of an upper body part of a child or toddler on a mattress upward and downward. Additionally, the user may choose to activate and/or deactivate the speaker unit 16, but might also control other aspects of the device.

In a system according to the disclosure, the control unit can comprise artificial intelligence i.e. be self-learning. For example the system can use the one or more sensors 17 to gather data about movements and movement patterns of a child or toddler and noise, such as for example crying, and for example correlations there between, in order to learn to set thresholds and/or to set and/or adjust the pump for setting the movement pattern of the at least one plank, for example in amplitude, frequency, fitting and the like. The system can for example also gather data on under what circumstances the system is switched on and/or off by a carer, to learn to anticipate and automatically perform such functions and/or warn a carer accordingly.

In embodiments a lifting unit 6 for use in a device 1 and/or a system may comprise a bladder 7 and a control unit 8, wherein the control unit 8 comprises an air pump 64 connected or connectable to the bladder 7 through at least one tube. The control unit 8 is configured to inflate and/or deflate the bladder 7 according to a programmed rhythm by activating the air pump 64. The rhythm may be chosen by a user or may be preset. The control unit 8 can further be arranged for activating the pump 64 based on a signal received from a sensor sensing a body-related parameter or a change is such body-related parameter.

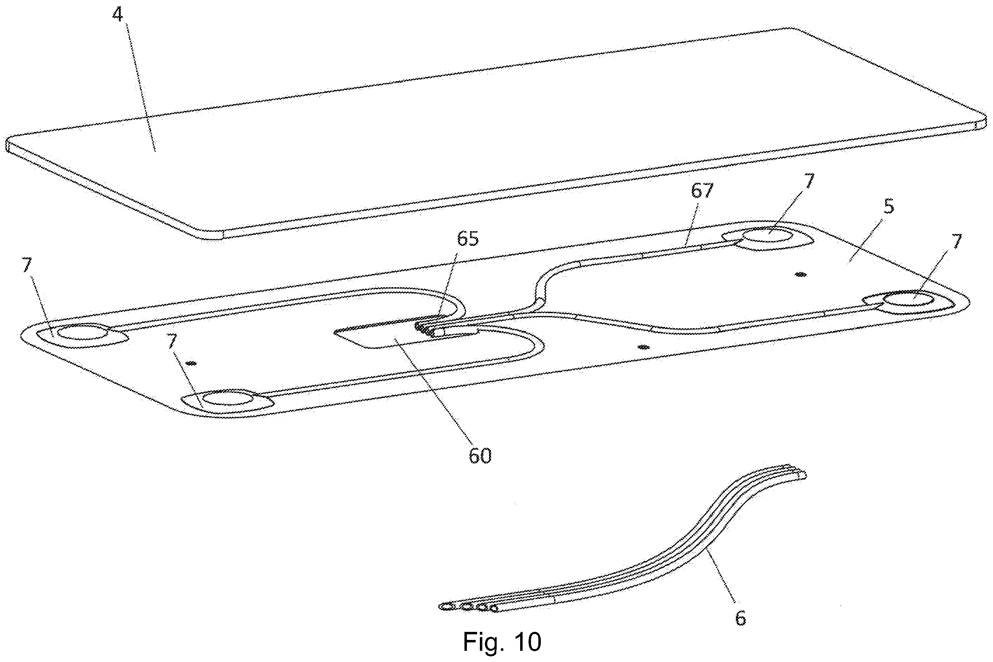

In the embodiment of Fig. 10 the at least one support 5 can be a plank, wherein the plank can comprise a closeable opening 60 for wires and/or tubes. This allows wires, tubes and / or hoses arranged between the pump system P and the at least one bladder 7 to be connected from below the child's bed 12. Such opening could obviously also be provided in a different position, for example facing sideways.

In advantageous embodiments, as discussed with reference to e.g. fig. 1, the lifting unit 6 can comprise at least four bladders 6, of which two bladders 7 can be provided at a head end 61 of the cot or child's bed and two bladders can be provided at a feet end 63 of the bed 12 during use. The bladders 7 can be provided in the corners of the at least one support 5. Each bladder 7 can be individually connected to the pump system P via a corresponding tube 13, allowing for the individual inflation of each bladder 7 when a pump 64 or pump chamber is connected to the corresponding tube 13. Preferably for each bladder 7 the system comprises a pump chamber or pump.

In order to facilitate customization of different types of movement caused by the inflating and deflating of each individual bladder 7, a distribution unit 65 can be provided in embodiments. The distribution unit 65 can be arranged to selectively connect pump chambers of the pump 64 to a bladder 7. In the shown embodiment, the distribution unit 65 can be provided as an releasable connection provided in the tube 13, effectively allowing the tube 13 to be separated in two components, a first hose part 66 and a second hose part 67. By connecting the second hose part 67 to the first hose part 66 of a bladder 7 via the releasable connection, each pump 64 of the pump system P can be connected to any one of the bladders 7. Different types of distribution units 65 can be used, for example a system with valves so that the hose parts 66,67 can be fixed such that no releasable connection is needed. Such a valve system can be operated by hand, or via the use of buttons or an app. In the same or further embodiments, the pump is connected to the bladder via the hose, and wherein the hose has a length such that the pump can be provided outside the bed when in use. In embodiments different bladders 7 can be connected to a same pump or pump chamber, or can be individually connected to different pumps or pump chambers.

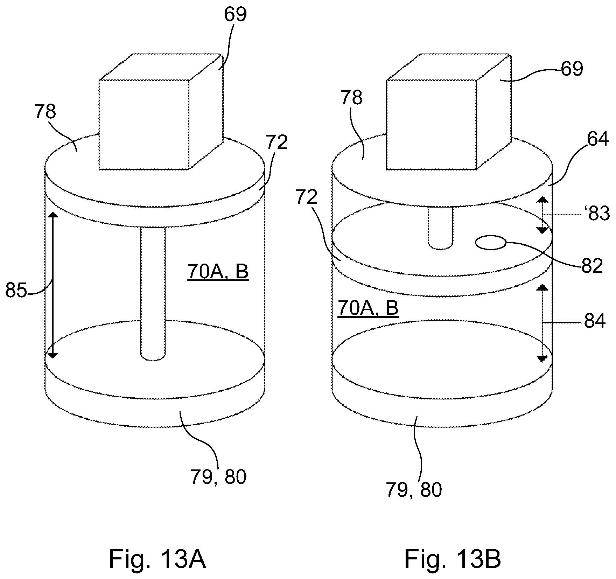

In the same or further embodiments, during use preferably a minimum pressure is maintained in each bladder 7, preferably such that the at least one plank 4 rests, or is carried, only by the bladders 7, even when the bladders are deflated. In other words, when the bladders 7 are deflated, there is still sufficient air present in each bladder 7 such that the at least one plank 4 does not rest on the at least one support 5. This can be achieved by not using the full range of motion of the pistons 72, or in other words, stop expanding the pump chamber 70 when there is still a desired volume of air and/or air pressure present in each bladder 7 as schematically shown in fig. 13B, wherein for building pre-pressure the piston 72 is moved down over a first distance 83 compared to the maximum distance 85 between the piston 72 and the bottom 79, 80 as shown in fig. 13A, whereas by arrow 84 the possible movement is indicated over which then the piston 72 can move up and down for deflating and inflating the bladder 7 connected there to such that the at least one plank 4 rests on each bladder 7.

Doing so allows for the use of pressure sensors 96 in one of the bladders or more bladders, for example in or for each bladder 7, even when the bladders are in a deflated state. By providing pressure sensors 17 for one, some or each bladder and maintaining a minimum pressure in each bladder 7, changes in pressure inside the bladder or bladders can be monitored, which can for example result from repositioning or other movements of the child in the bed, which can for example be an indication of the child starting to wake up or being uncomfortable, which indication can for example be used to (re)activate the system and/or change a pattern of inflating and deflating the bladders 7.

Moreover, by maintaining a minimum pressure, also referred to as pretension when deflated, in the bladders, surprisingly the responsiveness of the system can be improved even further, and for example a more continuous rocking motion can be obtained during use. The system can for example be set to have a standard pretension in said bladders during use. Alternatively the system can be designed to allow for setting a pretension, for example but not limited to based on the weight of the baby or toddler, measurements of pressure and pressure changes during use of the system, desired responsiveness or the like.

In a device according to the disclosure preferably a skirt 68 is provided which at least during use prevents that a person, especially a baby or toddler, can easily insert a body part, such as for example a finger, hand, toe or foot between the plank 4 and the support 5, or between an edge of the plank 4 and a side of the bed. The skirt is pliable enough to allow the desired movement of the plank 4 relative to the support 5, but rigid enough and/or pretensioned outward enough not to be allowed to be pushed in easily or accidentally between the plank and the support by a child or toddler by his or her hands or feet.

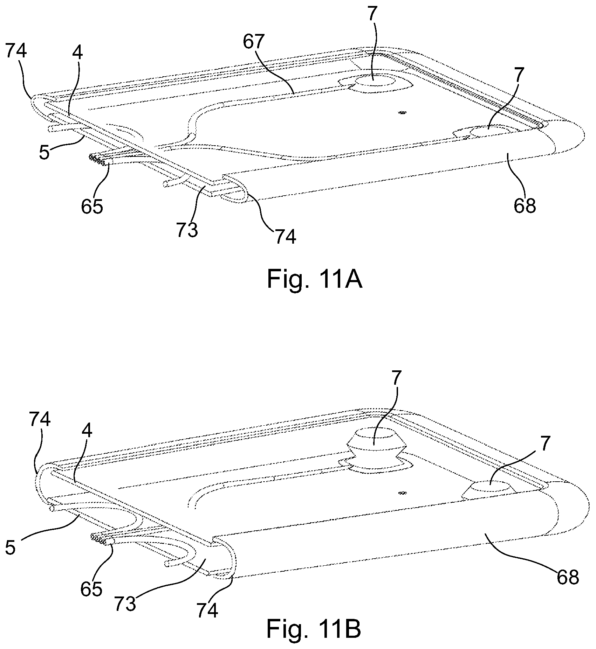

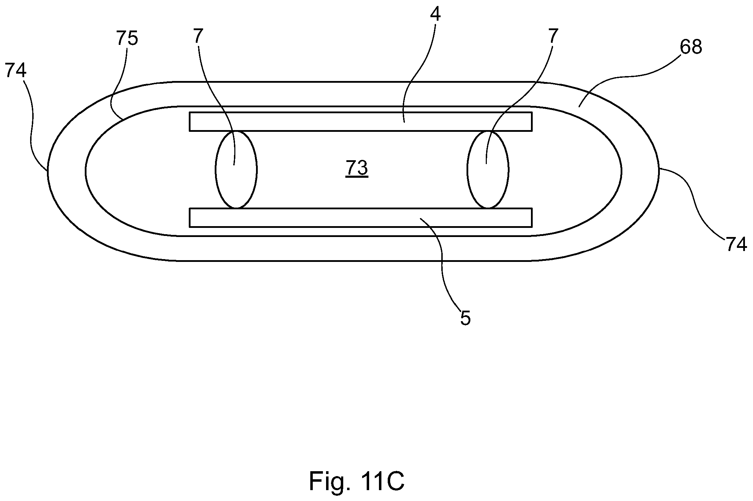

In the embodiments of the disclosure such as for example the embodiments shown in Figs. 11A, 11B and 11C the skirt 68 is shown as a pliable skirt 68 which extends from, more specifically can be connected to, the circumference of the at least one plank 4 and can be attached to the circumference of the at least one support 5. More specifically, the skirt 68 can extend around the perimeter of the plank 4 and support 5 and preferably forms a closed loop, preventing access to the space 73 between the at least one plank 4 and the at least one support 5. Such skirt 68 can be used in any of the embodiments shown and described. A skirt should in this disclosure be understood as meaning at least a flexible and/or movable closure of a space 73 between the at least one plank 4 and the at least one support 5 along at least part of the periphery of the said plank and/or support, preferably the entire periphery. In embodiments a skirt 68 can be a pliable element such as a band or strip, which preferably is elastically deformable for adjusting to a varying distance between the at least one plank 4 and the at least one support 5, and which bulges outward from said space 73, at least when reducing the distance between the plank 4 and support 5, preferably in all positions.

A skirt 68 for use in the present invention preferably has a generally substantially C-shaped cross section, as for example schematically shown in fig. 11, or for example a substantially D-shaped cross section as shown in fig. 9, such that an apex 74 of the skirt 68 will be biased outward from the space 73 and during use will be forced towards and preferably against an inside of at least the sides 26 of the bed 12, and preferably also against the head board 15 and the foot board 25 of the bed. In such embodiments the skirt 68 will not only prevent for example a child lying in the bed from getting body parts such as fingers getting caught between the plank 4 and the support 5, but also will reduce the risk that a person from outside the bed, or the baby or toddler himself or herself will introduce anything between the plank 4 and the support 5, such as for example but not limited to body parts such as fingers, for example when the bed 1 has partly open sides 26 and/or head board 15 and/or foot board 24, as shown in fig. 1.

When the bladders 7 are inflated, as shown in figure 11B, the skirt 68 can span the space 73 between the at least one plank 4 and the at least one support 5. When the bladders are deflated, as shown in figure 11A, the skirt 68 can extend elastically in an outward direction, away from the space 73 between the at least one plank 4 and the at least one support 5. Preferably the skirt will be designed such that during use of the device the skirt will be biased in outward direction, such that it will engage and preferably be pushed against an inner side of the bed sides as discussed.

Different types of materials can be used for the skirt 68, such as for example a rubber or elastomeric profile or a foam, or cloth provided with a plastic frame that allows for the above described flexibility, pliability and/or movement. It will be clear that a skirt 68 can also be provide in a different manner, such as for example but not limited to a skirt mounted to a peripheral edge of the at least one plank, hanging down alongside the sides of the space 73, passed the support 5, such that the skirt moves up and down alongside the support 5 with movement of the plank 4. A skirt 68 could also be formed by a bellow type of closure of the space 73, or by two parts, one mounted to the plank and another mounted to the support, said parts moving alongside each other when moving the plank relative to the support. Also a skirt 68 could be inflatable for providing the desired outward bias.

In embodiments the skirt 68 can be provided as part of a cover 75 enclosing the device, including the at least one plank 4, the at least one support 5 and the bladder or bladders 7. Such cover 75 can for example be made of fabric, and can be made to be removable from the further support device 2, for example for being cleaned, such as washed. The cover 75 can to that end for example be comparable to a mattress cover and can for example be provided with closing means such as a zipper or Velcro, for allowing access into the cover 75 for placing and removal of the further device 2.

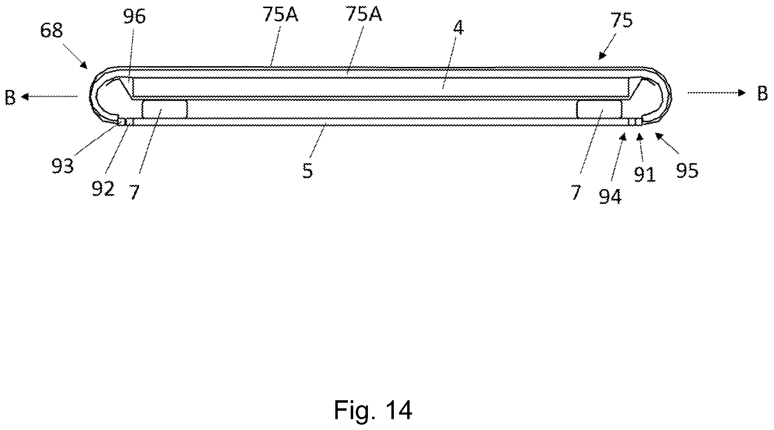

In preferred embodiments, as for example schematically shown in fig. 14, a device 1 comprises a plank 4 and a support 5, wherein at least the plank 4 is relatively stiff, as discussed, whereas the support 5 can for example be relatively flexible, at least as compared to the plank 4. In this embodiment four bladders 7 are provided, as discussed, one in or near each corner between the plank 4 and the support 5. Especially in relatively small embodiments also fewer bladders 7 can be provided, for example two. The support 5 is provided, along it periphery 94, with a first connecting means, such as for example one part 92 of a zipper 91. A cover 75 is provided, comprising an outer layer 75A of fabric, covering a layer 75B of a flexible, resilient material, such as a foam, for example but not limited to PU foam, PE foam, latex or the like. The cover 75 has a peripheral edge 95 which is provided with a second connecting means, here shown as a second part 93 of said zipper 91, such that the cover 75 can be provided over the plank 4 and can be connected to the support 5 by closing the zipper 91. Preferably a cover 75 has a pocket 96 below the outer layer 75A, preferably also below the layer 75B, into which the plank 4 can be inserted. In such embodiment the bladders 7 can for example be connected to the part of said pocked 96 facing downward, or only be connected to the support 5, the plank 4 inside the pocket 96 resting on the bladders 7. The layer 75B is made such that when the peripheral edge 95 is folded over the edges of the plank 4 to be connected to the support 5, the skirt 68 will be formed by the layers 75A, 75B, resiliently biased outward in a direction B substantially parallel to top surface of the plank 4. A skirt can also be provided differently, for example connected directly to the plank and/or support, with or without a cover.

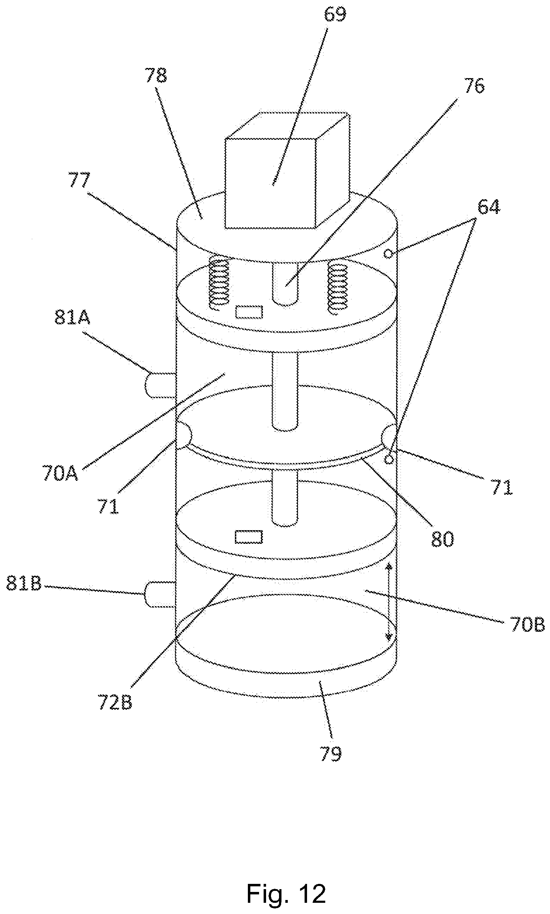

As shown in the embodiment of Fig. 12 the pump system P can comprise one motor 69 arranged to engage two pump chambers 70A, 70B of the pump 64. The system can for example have four such chambers, one for each bladder. The pump chambers 70 can be substantially airtightly separated, for example via a rolling diaphragm 71. However a seal can also be used, in combination with a piston 72, for example forming a piston with a piston ring as known in the art. In the same or a different embodiment, each chamber 70A, 70B may be provided with an over- and/or under pressure valve 82. The valve 82 can selectively provide a connection of the surrounding of the pump system P with a corresponding chamber 70A, 70B, in case the pressure in the corresponding pump chamber is too high, i.e. an over pressure, or if the pressure in the corresponding pump chamber is too low, i.e. near vacuum conditions.

In the embodiment shown a single motor 69 drives a piston rod 76, the rod supporting two pistons 72 within a cylinder 77, which is closed off at an upper end 78 and a lower end 79. Moreover between the upper and lower ends a further closing element 80 is provided. The rod extends though the upper end closure 77 and through the further closure 80, such that the pistons 72 can be moved up and down simultaneously. A first pump chamber 70A is formed between the upper piston 72A and the further closure 80, whereas a second pump chamber 70B is formed between the lower piston 72B and the bottom closure 79. Each pump chamber 70A, 70B has its own connector 81, for attachment of a tube or hose 66, 67 as discussed, connecting the pump chamber 70 to at least one bladder 7, preferably to a single bladder 7. In this embodiment each pump chamber 70 is, at least during use, airtight, except for the connector 81, such that with the or each bladder 7 connected to the relevant pump chamber 70 a closed circuit is formed. Thus by compressing the pump chamber 70 the air is forced from the pump chamber through the hose 66, 9 into a bladder 7, inflating it, whereas when the piston 72 is pulled up again, expanding the relevant chamber or chambers 70, the air is sucked out of the bladder 7, back into the chamber 70. Preferably the spaces within the cylinder 77 separated from the pump chambers 70 are in open connection with the environment, in order to substantially avoid pressure build up in these spaces.

In the same or other embodiments, one or more springs 82 can be provided between e.g. the piston 72 and the upper end 78, lower end 79 or the further closure 80. Using the spring 82, it can be possible to store a part of the kinetic energy during a movement in which the single motor 69 uses a relative small amount of power, which is the downward motion of the at least one plank 4, when the bladders 7 are deflated, and use release the energy to aid the single motor 69 when a relative large amount of power is needed, which is the upward motion of the at least one plank 4, or when the bladders 7 are inflated. The spring can for example be a compression spring compressed when the pump chambers are expanded, and releasing said energy when reducing the volume of the chambers 70. The spring can thus especially aid in overcoming first resistance against moving of the piston.

As discussed with reference to fig. 13, the pump can be driven such that during use at all times a volume of air is maintained in the or each bladder 7, such that pressure changes can be detected and the responsiveness of the system 1 is increased.

It will be clear that in the same way a single pump chamber can be operated by a single motor, or more than two pump chambers can be operated by a single motor.

As an alternative, the single piston can drive air in both pump chambers, resulting in an anti-phase pumping cycle. In other words, when in a first pump chamber air is compressed to form an over pressure such that the air is forced towards a corresponding bladder, the second pump chamber has an under pressure, forcing air from a corresponding bladder in to the pumping chamber. In embodiments, the pump system can be arranged to form a substantially closed system with the connected bladders such that during inflation of the bladders an amount of air can be transported towards the bladders that is substantially equal to the amount of air that can be returned to the pump system during deflation of the bladders.

In embodiments a plank can be combined with bladders as discussed, without a skirt, or a skirt can be provided with a more flexible plank and bladders as discussed,

The invention is not in any way limited to the exemplary embodiments shown in the description and the drawings. All combinations of (parts of) embodiments described and/or shown as well as many variations thereon are possible if falling within the scope of the invention as set forth in the appended claims.

## Claims

1. A device (1) for a cot or child's bed to encourage sleep, comprising:
a support device (2) to be placed in or under a mattress (3), comprising at least
at least one plank (4), and
at least one support (5),
a lifting unit (6), wherein the lifting unit (6) comprises at least one bladder (7), preferably at least two bladders (7), positioned between the at least one plank (4) and the at least one support (5),
the lifting unit (6) further comprising a control unit (8), wherein the control unit (8) is configured to inflate and/or deflate the at least one bladder (7) via a pump of a pump system (P) for providing an alternating upward and downward motion of the at least one plank,
a pliable skirt (68) extending at least outward from a circumference of the at least one plank (4), wherein the plank is stiff, such that if the plank is supported at two opposite longitudinal ends (61, 63) thereof, in a horizontal position and a vertical force of 150 N is applied to a middle of the plank (4), the middle of the plank (4) will be pushed down no more than 10 % of the distance between the opposite ends (61, 63), preferably no more than 6%, more preferably no more than 4%, more preferably no more than 3%, even more preferably no more than 2%, such as for example between 0 and 1.5%.

2. A device according to claim 1, wherein the skirt (68) is connected to the circumference of the one plank (4) and/or is connected to a circumference of the at least one support (5), preferably to the circumference of both the plank (4) and the support (5) and/or wherein the skirt (68) is part of a cover (75) extending over the plank (4) and wherein the skirt (68) comprises part of the cover (75) elastically shaped over a peripheral edge of at least the plank (4), such that the said part of the cover biases the cover outward, forming the skirt (68), wherein the plank (4) is stiff, such that it substantially does not deform during use.

3. A device according to any of the preceding claims, wherein the skirt (68) is suspended from the plank (4), and/or wherein the skirt (68) extends around the perimeter of the plank (4) forming a closed loop, and/or wherein the skirt (68) extends elastically in at least an outward direction, wherein the skirt (68) preferably is biased in said outward direction.

4. A device according to any one of the preceding claims, wherein the support (5) is a further plank (4), comprising a closeable opening (60) for wires and / or tubes.

5. A device according to any of the preceding claims, wherein the lifting unit (76) comprises at least four bladders (7), and wherein at least one bladder (7) is provided at a head-end (61) of the cot or child's bed during use, and/or wherein each bladder (7) is individually inflatable by a single pump.

6. A device according to any of the preceding claims, wherein the pump system (P) comprises a distribution unit (65), arranged to selectively connect the pump (64) of the pump system (P) to at least one bladder (7), and/or wherein the pump (64) is connected to the at least one bladder (7) via a hose, wherein the hose between the pump (64) and the at least one bladder (7) has a length such that the pump (64) can be provided outside of the cot or child's bed (12) when in use, and/or wherein the pump (64) comprises at least one pump chamber (70) and at least one spring (82), wherein the at least one spring (82) is arranged to store kinetic energy when the pump chamber (70) expands and to release kinetic energy when the pump chamber (70) contracts, and/or wherein the pump system (P) comprises at least one motor arranged to engage at least two pump chambers (70) of the pump (64).

7. A device according to any of the preceding claims, wherein the pump system (P) is arranged to form a substantially closed system with the at least one bladder (7) such that during inflation of the at least one bladder (7) an amount of air is transported from the pump system (P) towards the at least one bladder (7) that is substantially equal to the amount of air transported from the at least one bladder (7) to the pump system (P) during deflation of the at least one bladder (7) and/or wherein during use at least a minimum pressure is maintained in the or each bladder (7) when deflated and/or wherein at least one pressure sensor (17) is provided for during use sensing of pressure in at least one bladder (7).

8. A device according to any of the preceding claims, further comprising a sensor unit (9) arranged for detecting change in a body-related parameter of a child, wherein the sensor unit (9) further is configured to activate the control unit (8) when detecting the change in the body-related parameter , wherein the sensor unit (9) preferably comprises a sensor element for sensing a change in the body-related parameter, wherein the body-related parameter is at least one of:
- a respiratory rate of the child
- a heart rate of the child
- sound produced by the child
- position of the child
- movement of the child, wherein more preferably the or each sensor element (17) is in direct fluidum connection with a pumping chamber (70) of the pump system (P), and/or , wherein the at least one sensor element (17) is placed in and/or on the lifting unit (6) and/or the support device (2).

9. A device according to any one of the preceding claims, wherein the device (1) is configured to deactivate after a predetermined time period after activation, and/or wherein the device (1) is configured to activate when a change in the body-related parameter exceeds a predefined threshold value and configured to deactivate when the change in the body-related parameters comes below a predefined threshold value.

10. A device according to any of the preceding claims, wherein the support device (2) comprises a left portion (18, 19) and a right portion (20, 21), wherein the left portion (18, 19) and the right portion (20, 21) are adjustable relative to each other in a direction perpendicular to the direction in which the at least one plank (4) moves relative to the at least one support (5) for adaption of the width of the support device (2), and/or wherein the at least one plank (4) is hingeably connected to the at least one support (5), and/or wherein the at least one plank (4) is arranged for hinging upward about a hinging axis (a1) and the at least one support (5) comprises a first portion extending from the hinging axis (a1) in a first direction (d3) that is perpendicular to the hinging axis (a1) to below the at least one plank (4), and a second portion extending from the hinging axis (a1) in a direction (d4) opposite to the first direction (d3).

11. A device according to any of the claims 9 - 10, comprising a speaker unit (16) configured for generating a sound for a predetermined time period upon activation of the control unit by the sensor unit.

12. A device (1) according to any of the preceding claims, wherein the device (1) is a portable device (1).

13. A device according to any one of the preceding claims, wherein the system is designed such that the lifting unit (6) can be held in a lifting position over a period of time, such that the at least one plank (4) can be held in a tilted upward position for a period of time, wherein during said period of time preferably the lifting unit (6) can be operated for reciprocally lifting and lowering the at least one plank (4), such that even during such reciprocal inflation and deflation the at least one plank (4) stays tilted.

14. A mattress in which the device according to any of the preceding claims has been incorporated.

15. A method for encouraging sleep of a child, using a device comprising a support device, a control unit and a lifting unit, according to any one of claims 1 - 13, said method comprising the steps of:
positioning the support device on, in and/or under a mattress;
wherein the method further comprises the steps of:
activating the control unit for controlling the lifting unit for alternatingly moving at least part of an upper body part of the child upward and downward; and/or the step of
deactivating the control unit,
wherein the device further preferably comprises a sensor unit, and said method preferably further comprises the steps of:
sensing a body related parameter of a child on the device, and generating a signal based on the sensed body related parameter and/or change therein;
receiving and analyzing said signal;
generating an activation signal for the control unit if a predetermined threshold value has been exceeded;
receiving the signal; and / or the steps of
sensing a body related parameter of the child, and generating a signal based on the sensed body related parameter and/or change therein;
receiving and analyzing said signal;
generating a deactivation signal for the control unit if coming below a predetermined threshold value;
receiving the deactivation signal.

## Patentansprüche

1. Vorrichtung (1) für ein Kinderbett oder Kinderbettchen zur Förderung des Schlafs, umfassend:
eine Stützvorrichtung (2), die in oder unter einer Matratze (3) anzuordnen ist, umfassend zumindest
mindestens eine Platte (4) und
mindestens eine Stütze (5),
eine Hubeinheit (6), wobei die Hubeinheit (6) mindestens eine Blase (7), vorzugsweise mindestens zwei Blasen (7), umfasst, die zwischen der mindestens einen Platte (4) und der mindestens einen Stütze (5) positioniert ist bzw. sind,
wobei die Hubeinheit (6) ferner eine Steuereinheit (8) umfasst, wobei die Steuereinheit (8) dazu konfiguriert ist, die mindestens eine Blase (7) über eine Pumpe eines Pumpensystems (P) aufzublasen und/oder zu entleeren, um eine abwechselnde Aufwärts- und Abwärtsbewegung der mindestens einen Platte bereitzustellen,
eine biegsame Schürze (68), die sich zumindest von einem Umfang der mindestens einen Platte (4) nach außen erstreckt, wobei die Platte steif ist, sodass, wenn die Platte an ihren zwei gegenüberliegenden Längsenden (61, 63) in einer horizontalen Position abgestützt ist und eine vertikale Kraft von 150 N auf eine Mitte der Platte (4) ausgeübt wird, die Mitte der Platte (4) um nicht mehr als 10 % des Abstands zwischen den gegenüberliegenden Enden (61, 63), vorzugsweise um nicht mehr als 6 %, bevorzugter um nicht mehr als 4 %, bevorzugter um nicht mehr als 3 %, noch bevorzugter um nicht mehr als 2 %, wie beispielsweise zwischen 0 und 1,5 %, nach unten gedrückt wird.

2. Vorrichtung nach Anspruch 1, wobei die Schürze (68) mit dem Umfang der einen Platte (4) verbunden ist und/oder mit einem Umfang der mindestens einen Stütze (5) verbunden ist, vorzugsweise mit dem Umfang sowohl der Platte (4) als auch der Stütze (5), und/oder wobei die Schürze (68) Teil eines Bezugs (75) ist, der sich über die Platte (4) erstreckt, und wobei die Schürze (68) einen Teil des Bezugs (75) umfasst, der elastisch über einen Umfangsrand zumindest der Platte (4) geformt ist, sodass der genannte Teil des Bezugs den Bezug nach außen vorspannt und die Schürze (68) bildet, wobei die Platte (4) steif ist, sodass sie sich während des Gebrauchs im Wesentlichen nicht verformt.

3. Vorrichtung nach einem der vorhergehenden Ansprüche, wobei die Schürze (68) an der Platte (4) aufgehängt ist und/oder wobei sich die Schürze (68) um den Umfang der Platte (4) erstreckt und eine geschlossene Schleife bildet und/oder wobei sich die Schürze (68) zumindest in einer Richtung nach außen elastisch erstreckt, wobei die Schürze (68) vorzugsweise in die genannte Richtung nach außen vorgespannt ist.

4. Vorrichtung nach einem der vorhergehenden Ansprüche, wobei die Stütze (5) eine weitere Platte (4) ist, die eine verschließbare Öffnung (60) für Drähte und/oder Schläuche umfasst.

5. Vorrichtung nach einem der vorhergehenden Ansprüche, wobei die Hubeinheit (76) mindestens vier Blasen (7) umfasst und wobei während des Gebrauchs mindestens eine Blase (7) an einem Kopfende (61) des Kinderbetts oder Kinderbettchens vorgesehen ist und/oder wobei jede Blase (7) durch eine einzelne Pumpe individuell aufblasbar ist.

6. Vorrichtung nach einem der vorhergehenden Ansprüche, wobei das Pumpensystem (P) eine Verteilereinheit (65) umfasst, die dazu angeordnet ist, die Pumpe (64) des Pumpensystems (P) selektiv mit mindestens einer Blase (7) zu verbinden, und/oder wobei die Pumpe (64) über einen Schlauch mit der mindestens einen Blase (7) verbunden ist, wobei der Schlauch zwischen der Pumpe (64) und der mindestens einen Blase (7) eine derartige Länge aufweist, dass die Pumpe (64) während des Gebrauchs außerhalb des Kinderbetts oder Kinderbettchens (12) vorgesehen werden kann, und/oder wobei die Pumpe (64) mindestens eine Pumpenkammer (70) und mindestens eine Feder (82) umfasst, wobei die mindestens eine Feder (82) dazu angeordnet ist, kinetische Energie zu speichern, wenn sich die Pumpenkammer (70) ausdehnt, und kinetische Energie freizusetzen, wenn sich die Pumpenkammer (70) zusammenzieht, und/oder wobei das Pumpensystem (P) mindestens einen Motor umfasst, der dazu angeordnet ist, mit mindestens zwei Pumpenkammern (70) der Pumpe (64) in Eingriff zu treten.

7. Vorrichtung nach einem der vorhergehenden Ansprüche, wobei das Pumpensystem (P) dazu angeordnet ist, mit der mindestens einen Blase (7) ein im Wesentlichen geschlossenes System zu bilden, sodass während des Aufblasens der mindestens einen Blase (7) eine Luftmenge von dem Pumpensystem (P) zu der mindestens einen Blase (7) transportiert wird, die im Wesentlichen gleich der Luftmenge ist, die während des Entleerens der mindestens einen Blase (7) von der mindestens einen Blase (7) zu dem Pumpensystem (P) transportiert wird, und/oder wobei während des Gebrauchs in der oder jeder Blase (7) im entleerten Zustand mindestens ein Mindestdruck aufrechterhalten wird und/oder wobei mindestens ein Drucksensor (17) zum Erfassen des Drucks in mindestens einer Blase (7) während des Gebrauchs vorgesehen ist.

8. Vorrichtung nach einem der vorhergehenden Ansprüche, ferner umfassend eine Sensoreinheit (9), die zum Erfassen einer Änderung eines körperbezogenen Parameters eines Kindes angeordnet ist, wobei die Sensoreinheit (9) ferner dazu konfiguriert ist, die Steuereinheit (8) bei Erfassung der Änderung des körperbezogenen Parameters zu aktivieren, wobei die Sensoreinheit (9) vorzugsweise ein Sensorelement zum Erfassen einer Änderung des körperbezogenen Parameters umfasst, wobei der körperbezogene Parameter mindestens einer der folgenden ist:
- eine Atemfrequenz des Kindes
- eine Herzfrequenz des Kindes
- ein von dem Kind erzeugtes Geräusch
- eine Position des Kindes
- eine Bewegung des Kindes, wobei bevorzugter das oder jedes Sensorelement (17) in direkter Fluidverbindung mit einer Pumpenkammer (70) des Pumpensystems (P) steht und/oder wobei das mindestens eine Sensorelement (17) in und/oder an der Hubeinheit (6) und/oder der Stützvorrichtung (2) angeordnet ist.

9. Vorrichtung nach einem der vorhergehenden Ansprüche, wobei die Vorrichtung (1) dazu konfiguriert ist, sich nach einem vorbestimmten Zeitraum nach der Aktivierung zu deaktivieren, und/oder wobei die Vorrichtung (1) dazu konfiguriert ist, sich zu aktivieren, wenn eine Änderung des körperbezogenen Parameters einen vordefinierten Schwellenwert überschreitet, und dazu konfiguriert ist, sich zu deaktivieren, wenn die Änderung der körperbezogenen Parameter einen vordefinierten Schwellenwert unterschreitet.

10. Vorrichtung nach einem der vorhergehenden Ansprüche, wobei die Stützvorrichtung (2) einen linken Abschnitt (18, 19) und einen rechten Abschnitt (20, 21) umfasst, wobei der linke Abschnitt (18, 19) und der rechte Abschnitt (20, 21) relativ zueinander in einer Richtung senkrecht zu der Richtung, in der sich die mindestens eine Platte (4) relativ zu der mindestens einen Stütze (5) bewegt, verstellbar sind, um die Breite der Stützvorrichtung (2) anzupassen, und/oder wobei die mindestens eine Platte (4) schwenkbar mit der mindestens einen Stütze (5) verbunden ist und/oder wobei die mindestens eine Platte (4) zum Aufwärtsschwenken um eine Schwenkachse (a1) angeordnet ist und die mindestens eine Stütze (5) einen ersten Abschnitt, der sich von der Schwenkachse (a1) in einer ersten Richtung (d3), die senkrecht zu der Schwenkachse (a1) verläuft, bis unter die mindestens eine Platte (4) erstreckt, und einen zweiten Abschnitt umfasst, der sich von der Schwenkachse (a1) in einer der ersten Richtung (d3) entgegengesetzten Richtung (d4) erstreckt.

11. Vorrichtung nach einem der Ansprüche 9-10, umfassend eine Lautsprechereinheit (16), die dazu konfiguriert ist, bei Aktivierung der Steuereinheit durch die Sensoreinheit für einen vorbestimmten Zeitraum ein Geräusch zu erzeugen.

12. Vorrichtung (1) nach einem der vorhergehenden Ansprüche, wobei die Vorrichtung (1) eine tragbare Vorrichtung (1) ist.

13. Vorrichtung nach einem der vorhergehenden Ansprüche, wobei das System derart ausgelegt ist, dass die Hubeinheit (6) über einen Zeitraum in einer Hubposition gehalten werden kann, sodass die mindestens eine Platte (4) über einen Zeitraum in einer nach oben geneigten Position gehalten werden kann, wobei während des genannten Zeitraums vorzugsweise die Hubeinheit (6) zum wechselseitigen Anheben und Absenken der mindestens einen Platte (4) betrieben werden kann, sodass die mindestens eine Platte (4) selbst während eines derartigen wechselseitigen Aufblasens und Entleerens geneigt bleibt.

14. Matratze, in die die Vorrichtung nach einem der vorhergehenden Ansprüche integriert ist.

15. Verfahren zur Förderung des Schlafs eines Kindes unter Verwendung einer Vorrichtung, die eine Stützvorrichtung, eine Steuereinheit und eine Hubeinheit gemäß einem der Ansprüche 1-13 umfasst, wobei das genannte Verfahren die folgenden Schritte umfasst:
Positionieren der Stützvorrichtung auf, in und/oder unter einer Matratze; wobei das Verfahren ferner die folgenden Schritte umfasst:
Aktivieren der Steuereinheit zum Steuern der Hubeinheit, um zumindest einen Teil eines Oberkörperteils des Kindes abwechselnd nach oben und nach unten zu bewegen; und/oder den Schritt des
Deaktivierens der Steuereinheit, wobei die Vorrichtung ferner vorzugsweise eine Sensoreinheit umfasst und das genannte Verfahren vorzugsweise ferner die folgenden Schritte umfasst:
Erfassen eines körperbezogenen Parameters eines auf der Vorrichtung befindlichen Kindes und Erzeugen eines Signals auf Grundlage des erfassten körperbezogenen Parameters und/oder einer Änderung desselben;
Empfangen und Analysieren des genannten Signals;
Erzeugen eines Aktivierungssignals für die Steuereinheit, wenn ein vorbestimmter Schwellenwert überschritten wurde;
Empfangen des Signals; und/oder die Schritte des
Erfassens eines körperbezogenen Parameters des Kindes und Erzeugens eines Signals auf Grundlage des erfassten körperbezogenen Parameters und/oder einer Änderung desselben;
Empfangens und Analysierens des genannten Signals;
Erzeugens eines Deaktivierungssignals für die Steuereinheit, wenn ein vorbestimmter Schwellenwert unterschritten wird;
Empfangens des Deaktivierungssignals.

## Revendications

1. Dispositif (1) pour un lit de bébé ou d'enfant destiné à favoriser le sommeil, comprenant :
un dispositif de support (2) devant être placé dans ou sous un matelas (3), comprenant
au moins une planche (4), et
au moins un support (5),
une unité de levage (6), dans lequel l'unité de levage (6) comprend au moins une vessie (7), de préférence au moins deux vessies (7) positionnées entre l'au moins une planche (4) et l'au moins un support (5),
l'unité de levage (6) comprenant en outre une unité de commande (8), dans lequel l'unité de commande (8) est configurée pour gonfler et/ou dégonfler l'au moins une vessie (7) via une pompe d'un système de pompe (P) afin de permettre un mouvement alternatif vers le haut et vers le bas de l'au moins une planche,
une jupe (68) pliable s'étendant au moins vers l'extérieur à partir d'une circonférence de l'au moins une planche (4), dans lequel la planche est raide de sorte que si la planche est supportée au niveau de deux de ses extrémités (61, 63) longitudinales opposées dans une position horizontale et qu'une force verticale de 150 N est appliquée au milieu de la planche (4), le milieu de la planche (4) ne s'enfonce pas plus de 10 % de la distance entre les extrémités (61, 63) opposées, de préférence pas plus de 6 %, de manière davantage préférée pas plus de 4 %, de manière davantage préférée pas plus de 3 %, de manière encore plus préférée pas plus de 2 %, par exemple entre 0 et 1,5 %.

2. Dispositif selon la revendication 1, dans lequel la jupe (68) est connectée à la circonférence de la planche (4) et/ou est connectée à une circonférence de l'au moins un support (5), de préférence aux deux circonférences de la planche (4) et du support (5), et/ou dans lequel la jupe (68) fait partie d'un élément couvrant (75) s'étendant sur la planche (4), et dans lequel la jupe (68) comprend une partie de l'élément couvrant (75) formée de manière élastique sur un bord périphérique d'au moins la planche (4), de sorte que ladite partie de l'élément couvrant sollicite l'élément couvrant vers l'extérieur, formant la jupe (68), dans lequel la planche (4) est raide de sorte qu'elle ne se déforme pratiquement pas pendant l'utilisation.

3. Dispositif selon l'une des revendications précédentes, dans lequel la jupe (68) est suspendue à la planche (4), et/ou dans lequel la jupe (68) s'étend autour du périmètre de la planche (4) en formant une boucle fermée, et/ou dans lequel la jupe (68) s'étend élastiquement dans au moins une direction vers l'extérieur, de préférence dans lequel la jupe (68) est sollicitée dans ladite direction vers l'extérieur.

4. Dispositif selon l'une des revendications précédentes, dans lequel le support (5) est une planche (4) supplémentaire comprenant une ouverture refermable (60) destinée à des fils et/ou à des tubes.

5. Dispositif selon l'une des revendications précédentes, dans lequel l'unité de levage (76) comprend au moins quatre vessies (7), et dans lequel au moins une vessie (7) est prévue à une extrémité de tête (61) du lit de bébé ou d'enfant pendant l'utilisation, et/ou dans lequel chaque vessie (7) est gonflable individuellement par une seule pompe.

6. Dispositif selon l'une des revendications précédentes, dans lequel le système de pompe (P) comprend une unité de distribution (65), agencée pour connecter de manière sélective la pompe (64) du système de pompe (P) à au moins une vessie (7), et/ou dans lequel la pompe (64) est connectée à l'au moins une vessie (7) via un tuyau, dans lequel le tuyau entre la pompe (64) et l'au moins une vessie (7) a une longueur telle que la pompe (64) puisse être prévue à l'extérieur du lit de bébé ou d'enfant (12) en cours d'utilisation, et/ou dans lequel la pompe (64) comprend au moins une chambre de pompe (70) et au moins un ressort (82), dans lequel l'au moins un ressort (82) est agencé pour stocker de l'énergie cinétique lorsque la chambre de pompe (70) se dilate et pour libérer de l'énergie cinétique lorsque la chambre de pompe (70) se contracte, et/ou dans lequel le système de pompe (P) comprend au moins un moteur agencé pour impliquer au moins deux chambres de pompe (70) de la pompe (64).

7. Dispositif selon l'une des revendications précédentes, dans lequel le système de pompe (P) est agencé pour former un système sensiblement fermé avec l'au moins une vessie (7) de sorte qu'une quantité d'air transportée du système de pompe (P) vers l'au moins une vessie (7) pendant le gonflage de l'au moins une vessie (7) soit sensiblement égale à la quantité d'air transportée de l'au moins une vessie (7) vers le système de pompe (P) pendant le dégonflage de l'au moins une vessie (7), et/ou dans lequel, pendant l'utilisation, au moins une pression minimale est maintenue dans la vessie (7) ou dans chaque vessie (7) lorsqu'elle est dégonflée, et/ou dans lequel, pendant l'utilisation, au moins un capteur de pression (17) est prévu pour détecter la pression dans au moins une vessie (7).

8. Dispositif selon l'une des revendications précédentes, comprenant en outre une unité de capteur (9) agencée pour détecter un changement dans un paramètre corporel d'un enfant, dans lequel l'unité de capteur (9) est en outre configurée pour activer l'unité de commande (8) lorsqu'elle détecte le changement dans le paramètre corporel, dans lequel l'unité de capteur (9) comprend de préférence un élément de capteur pour détecter un changement dans le paramètre corporel, dans lequel le paramètre corporel est au moins un parmi :
- une fréquence respiratoire de l'enfant
- une fréquence cardiaque de l'enfant
- un son émis par l'enfant
- une position de l'enfant
- un mouvement de l'enfant, dans lequel, de manière davantage préférée, l'élément de capteur ou chaque élément de capteur (17) est en communication fluidique directe avec une chambre de pompage (70) du système de pompe (P), et/ou dans lequel l'au moins un élément de capteur (17) est placé dans et/ou sur l'unité de levage (6) et/ou le dispositif de support (2).

9. Dispositif selon l'une des revendications précédentes, dans lequel le dispositif (1) est configuré pour se désactiver après une période de temps prédéterminée suivant son activation, et/ou dans lequel le dispositif (1) est configuré pour s'activer lorsqu'un changement dans le paramètre corporel dépasse une valeur seuil prédéfinie et pour se désactiver lorsque le changement dans les paramètres corporels passe en dessous d'une valeur seuil prédéfinie.

10. Dispositif selon l'une des revendications précédentes, dans lequel le dispositif de support (2) comprend une partie gauche (18, 19) et une partie droite (20, 21), dans lequel la partie gauche (18, 19) et la partie droite (20, 21) sont réglables l'une par rapport à l'autre dans une direction perpendiculaire à la direction dans laquelle l'au moins une planche (4) se déplace par rapport à l'au moins un support (5), afin d'adapter la largeur du dispositif de support (2), et/ou dans lequel l'au moins une planche (4) est connectée de manière articulée à l'au moins un support (5), et/ou dans lequel l'au moins une planche (4) est agencée pour s'articuler vers le haut autour d'un axe d'articulation (a1), et l'au moins un support (5) comprend une première partie s'étendant depuis l'axe d'articulation (a1) dans une première direction (d3) perpendiculaire à l'axe d'articulation (a1) jusqu'en dessous de l'au moins une planche (4) et une deuxième partie s'étendant depuis l'axe d'articulation (a1) dans une direction (d4) opposée à la première direction (d3).

11. Dispositif selon l'une des revendications 9 et 10, comprenant une unité de haut-parleur (16) configurée pour générer un son pendant une période de temps prédéterminée à l'activation de l'unité de commande par l'unité de capteur.

12. Dispositif (1) selon l'une des revendications précédentes, dans lequel le dispositif (1) est un dispositif (1) portable.

13. Dispositif selon l'une des revendications précédentes, dans lequel le système est conçu de sorte que l'unité de levage (6) puisse être maintenue dans une position de levage pendant une période de temps de sorte que l'au moins une planche (4) puisse être maintenue dans une position inclinée vers le haut pendant une période de temps, de préférence dans lequel, pendant ladite période de temps, l'unité de levage (6) peut être actionnée pour soulever et abaisser mutuellement l'au moins une planche (4) de sorte que l'au moins une planche (4) reste inclinée même pendant ce gonflage et ce dégonflage mutuels.

14. Matelas dans lequel a été intégré le dispositif selon l'une des revendications précédentes.

15. Procédé pour favoriser le sommeil d'un enfant utilisant un dispositif comprenant un dispositif de support, une unité de commande et une unité de levage selon l'une des revendications 1 à 13, ledit procédé comprenant les étapes suivantes :
positionner le dispositif de support sur, dans et/ou sous un matelas ; dans lequel le procédé comprend en outre les étapes suivantes :
activer l'unité de commande pour commander l'unité de levage afin de déplacer alternativement au moins une partie du haut du corps de l'enfant vers le haut et vers le bas ; et/ou l'étape suivante
désactiver l'unité de commande,
de préférence dans lequel le dispositif comprend en outre une unité de capteur, et de préférence ledit procédé comprend en outre les étapes suivantes :
détecter un paramètre corporel d'un enfant sur le dispositif, et générer un signal sur la base du paramètre corporel détecté et/ou d'un changement de celui-ci ;
recevoir et analyser ledit signal ;
générer un signal d'activation pour l'unité de commande si une valeur seuil prédéterminée a été dépassée ;
recevoir le signal ; et/ou les étapes suivantes
détecter un paramètre corporel de l'enfant, et générer un signal sur la base du paramètre corporel détecté et/ou d'un changement de celui-ci ;
recevoir et analyser ledit signal ;
générer un signal de désactivation pour l'unité de commande dans le cas d'un passage en dessous d'une valeur seuil prédéterminée ;
recevoir le signal de désactivation.
